# EUROPÄISCHE PATENTSCHRIFT

(11) **EP 2 033 670 B1**
(45) Veröffentlichungstag und Bekanntmachung des Hinweises auf die Patenterteilung: **02.09.2015**
(21) Anmeldenummer: 07112624.7
(22) Anmeldetag: 17.07.2007
(51) Int. Cl.: A61B 19/00, A61L 31/12

(54) **Befestigungsmittel für medizinische Zwecke, insbesondere zum Befestigen einer Referenzgeometrie für navigationsunterstützte Operationen, an einem Körper, insbesondere an einem Knochen**
Attachment device for medical purposes, in particular for attaching a reference geometry for navigation-assisted operations to a body, in particular to a bone
Moyen de fixation pour objectifs médicaux, en particulier pour la fixation d'une géométrie de référence pour des opérations assistées par navigation, sur un corps, notamment sur un os

(43) Veröffentlichungstag der Anmeldung: 11.03.2009
(73) Patentinhaber: Brainlab AG, 85622 Feldkirchen (DE)
(72) Erfinder: Heigl, Rupert, 81379, München (DE); Lechner, Christian, 82287, Jesenwang (DE)
(74) Vertreter: Schwabe - Sandmair - Marx

(56) Entgegenhaltungen:
- EP-A- 0 171 884
- EP-A- 1 238 637
- EP-A1- 1 967 155
- GB-A- 2 405 342
- US-A1- 2005 065 515

## Beschreibung

### Gebiet der Erfindung

Die vorliegende Erfindung bezieht sich auf ein Befestigungsmittel für medizinische Zwecke, insbesondere zum Befestigen einer Referenzgeometrie für navigationsunterstützte Operationen an einem Körper, insbesondere an einem Knochen.

Das erfindungsgemäße Befestigungsmittel ist wenigstens teilweise aus einem bestimmten Material hergestellt, das Vorteile hinsichtlich Röntgentransparenz und 3D Röntgenbildartefakten bietet. Bevorzugt ist es so ausgebildet, dass es mittels eines bestimmten Mechanismus an einem Körper befestigt werden kann, der Vorteile hinsichtlich möglicherweise auftretenden Verspannungen und Verbiegungen bietet.

### Stand der Technik

Aus dem Stand der Technik sind bereits Befestigungsmittel für medizinische Zwecke, insbesondere zum Befestigen einer Referenzgeometrie für navigationsunterstützte Operationen, an einem Körper, insbesondere an einem Knochen, bekannt. Dabei handelt es sich um konstruktiv verschiedenartig ausgestaltete Befestigungsmittel wie zum Beispiel Klemmen bzw. Zangen, die mit spitzen Abschnitten wie zum Beispiel Spikes versehen sind, die bei der Befestigung an einem Körper bzw. Knochen in diesen hineingebohrt werden. Weitere bekannte Befestigungsmittel sind im Wesentlichen stabförmig ausgebildete Befestigungsmittel, in die Schrauben hineingedreht werden, damit spitze Abschnitte in den Körper bzw. Knochen zur Fixierung eindringen können. Es sind auch Befestigungsmittel in Form von Platten bekannt, die mittels mehrerer Schrauben an einem Körper, insbesondere an einem Knochen, befestigt werden.

Es ist im Bereich der navigationsunterstützen Chirurgie üblich, an den verschiedenen Befestigungsmitteln jeweils eine Referenzgeometrie zu befestigen. Darunter ist eine Vorrichtung mit sogenannten Markern zu verstehen, die bei navigationsunterstützten Operationen zum Einsatz kommen. Bei den Markern kann es sich um aktive Marker handeln, die selbst Strahlung emittieren, die von einer Kamera im Operationsraum erfasst wird, oder es kann sich um passive Marker handeln, die Strahlung lediglich reflektieren, wobei die reflektierte Strahlung dann von einem Kamerasystem im Operationsraum erfasst wird. Wird nun ein zu navigierendes Behandlungsgerät wie zum Beispiel ein chirurgisches Instrument ebenfalls mit einer Referenzgeometrie mit entsprechenden Markern versehen, und wird die Markereinrichtung des zu navigierenden Behandlungsgerätes zugleich mit der am Körper bzw. Knochen fixierten Markereinrichtung erfasst, so kann aus der ermittelten relativen Lage zwischen der Markereinrichtung am zu navigierenden Behandlungsgerät einerseits und der Markereinrichtung, die am Körper fixiert ist, andererseits, auf die Position des zu navigierenden Behandlungsgerätes rückgeschlossen werden.

Bei Operationen, die navigationsunterstützt durchgeführt werden, handelt es sich zumeist um Operationen, die mit höchster Präzision durchgeführt werden müssen. Aus diesem Grunde ist es wichtig, dass das Befestigungsmittel, an dem die Referenzgeometrie angebracht ist, sicher am Körper bzw. Knochen fixiert ist. Das Befestigungsmittel darf sich auf keinen Fall versehentlich lösen oder gar zerbrechen. Dabei ist zu bedenken, dass ein solches Befestigungsmittel sehr hohen mechanischen Beanspruchungen standhalten muss. Aus diesem Grunde sind herkömmliche Befestigungsmittel aus Edelstahl oder Titan gefertigt.

Eine weitere Anforderung an gattungsgemäße Befestigungsmittel ist ihre Sterilisierbarkeit. Die Befestigungsmittel werden nämlich mehrfach wiederverwendet.

Ein weiteres Kriterium für gattungsgemäße Befestigungsmittel ist ihre Größe. Je kleiner ein Befestigungsmittel ist, desto weniger ist es bei einer navigationsunterstützten Operation im Wege: Es behindert die Bewegungsfreiheit und Sichtfreiheit des Operateurs dann weniger.

Schließlich wäre es von Vorteil, wenn ein gattungsgemäßes Befestigungsmittel für medizinische Zwecke so hergestellt werden könnte, dass bei eventuell während der Operation durchzuführenden bildgebenden Verfahren keine oder nur geringe Artefakte entstünden, die verhindern, dass wichtige Details in den gewonnen Aufnahmen nicht zu erkennen sind. Bei den bildgebenden Verfahren kann es sich zum Beispiel um verschiedene Arten von Röntgenaufnahmen (2D-Röntgen, 3D-Röntgen oder 3D Computertomographie) handeln. In diesem Zusammenhang ist anzumerken, dass die Artefaktbildung bei zweidimensionalen Röntgenaufnahmen eine andere ist als bei dreidimensionalen Röntgenaufnahmen, die aus verschiedenen zweidimensionalen Aufnahmen zusammengesetzt und rekonstruiert werden. Werden Materialien verwendet, die röntgentransparent sind wie zum Beispiel Kunststoffe oder bestimmte Keramiken, so können im Allgemeinen gute zweidimensionale Röntgenaufnahmen (fluoroskopische Aufnahmen) erhalten werden. Dies bedeutet aber nicht automatisch auch, dass ein gutes dreidimensionales Bild bzw. gute zweidimensionale Aufnahmen, aus denen sich das 3D-Bild aufbaut, erhalten werden können, da hier auch bei Materialien mit besseren röntgentransparenten Eigenschaften Artefakte auftreten können. Entscheidend für das Artefaktverhalten ist nicht die Dichte des Materials, sondern vielmehr auftretende Dichteunterschiede in einem Körper und dessen Geometrie.

EP 1238 637 A1 offenbart ein längliches Implantat zum Befestigen an Knochen beidseits eines beschädigten Bereichs mittels einer Verbindungseinrichtung.

EP 1 718 848 A1 offenbart eine steife Endoprothese aus Polyaryletherketon.

WO 2004/075767 A1 offenbart eine Patella-Referenzvorrichtung mit einem an der Patella festlegbaren Grundkörper und einem detektierbaren Referenzelementelement sowie einer Verbindungsvorrichtung zum lösbaren Verbinden des Grundkörpers und des Referenzelements in unterschiedlichsten Referenzstellungen.

GB 2 405 342 A offenbart eine Knochenplatte aus Polyetheretherketon (PEEK).

EP 1 967 155 A1 offenbart eine medizinische Klemme, insbesondere eine Wirbelsäuleklemme, die mittels Drehung einer mittig angeordneten Rändelscheibe bewegbare Klemmschenkel aufweist.

### Beschreibung der Erfindung

Es ist die Aufgabe der vorliegenden Erfindung, ein verbessertes Befestigungsmittel für medizinische Zwecke, insbesondere zum Befestigen einer Referenzgeometrie für navigationsunterstützte Operationen, an einem Körper, insbesondere an einem Knochen wie zum Beispiel ein Dornfortsatz eines Wirbelköpers, bereitzustellen, das den oben aufgeführten Anforderungen möglichst gut gerecht wird.

Die Aufgabe wird gelöst durch den Gegenstand des unabhängigen Anspruches 1.

Abhängige Ansprüche sind auf bevorzugte Ausführungsformen gerichtet.

Das erfindungsgemäße Befestigungsmittel für medizinische Zwecke, insbesondere zum Befestigen einer Referenzgeometrie für navigationsunterstützte Operationen, an einem Körper, insbesondere an einem Knochen, ist dadurch gekennzeichnet, dass wenigstens ein Teil des Befestigungsmittels aus einer karbonfaserverstärkten unsubstituierten, karbonfaserverstärkten einfach substituierten oder karbonfaserverstärkten mehrfachsubstituierten Polyether-Verbindung, insbesondere aus einer karbonfaserverstärkten unsubstituierten, karbonfaserverstärkten einfach substituierten oder karbonfaserverstärkten mehrfachsubstituierten aromatischen Polyether-Verbindung besteht.

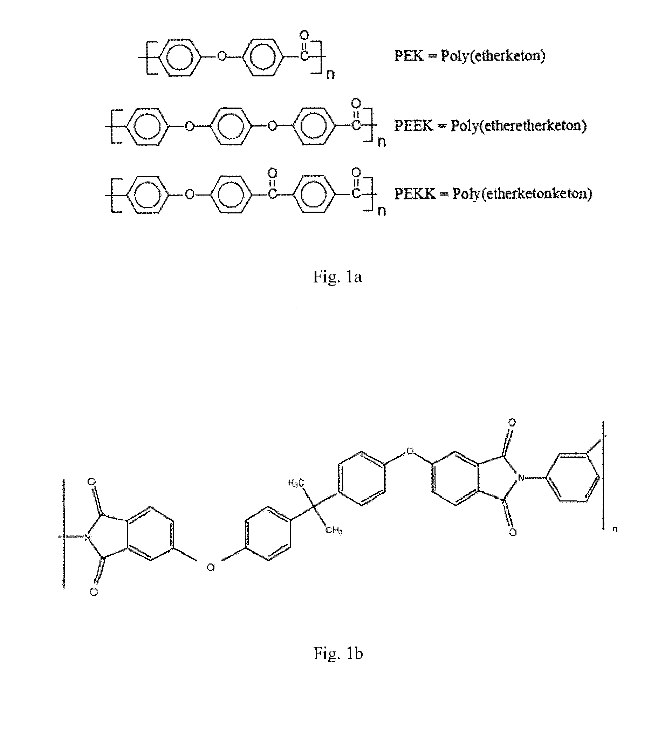

Bei der karbonfaserverstärkten unsubstituierten, karbonfaserverstärkten einfach substituierten oder karbonfaserverstärkten mehrfachsubstituierten aromatischen Polyether-Verbindung handelt es sich bevorzugt um karbonfaserverstärkte unsubstituierte, karbonfaserverstärkte einfach substituierte oder karbonfaserverstärkte mehrfachsubstituierte Polyaryletherketone, insbesondere um Poly(etheretherketon)e (PEEK), Poly(etherketon)e (PEK) oder Poly(etherketonketon)e (PEKK). Die Bedeutung bzw. das Zustandekommen der Bezeichnungen PEK, PEEK und PEKK am Markt soll im Folgenden kurz erklärt werden: Bei einem sogenannten Poly(etherketon) (PEK) ist die Hauptkette aus Benzolringen aufgebaut, die abwechselnd über eine Ether-Gruppe und über eine Keto-Gruppe miteinander verbunden sind. Dies ist in Figur 1a (oben) veranschaulicht. Bezogen auf die einzelnen Benzolringe erfolgt die Verbindung zur Kette jeweils in 1,4-Stellung, das heißt in para-Position. Die Verbindung der einzelnen Benzolringe innerhalb der Kette in para-Position verleiht den Poly(etherketon)en ihre verhältnismäßig große Festigkeit. Entsprechend wird unter PEEK ein Polymer verstanden, das sich aus mehreren Einheiten zusammensetzt, wobei jede Einheit aus drei Benzolringen besteht, die jeweils mittels einer ersten Ether-Brücke, einer zweiten Ether-Brücke und einer Keto-Brücke miteinander verbunden sind. Dies ist in Figur 1a (Mitte) veranschaulicht. Diese Systematik lässt sich auf für sogenanntes PEKK, das heißt Poly(etherketonketon) fortsetzen. Dabei handelt es sich um eine Vielzahl von Einheiten, in denen drei Benzolringe jeweils über eine Ether-Brücke, eine erste Keto-Brücke und eine zweite Keto-Brücke miteinander verbunden sind. Dies ist in Figur 1a (unten) dargestellt. Die Anzahl der in beschriebener Weise verknüpften Basiseinheiten ist in Figur 1 a mit n bezeichnet.

Gemäß einer weiteren bevorzugten Ausführungsform handelt es sich bei der karbonfaserverstärkten unsubstituierten, karbonfaserverstärkten einfach substituierten oder karbonfaserverstärkten mehrfachsubstituierten aromatischen Polyether-Verbindung um ein Polyetherimid (PEI), d.h. um ein Polymer, dessen Hauptkette aus über Ether- und Imid-Gruppen verknüpften aromatischen Ringen aufgebaut ist. Beispielhaft ist die Struktur eines solchen Polyetherimids in Figur 1b dargestellt. Die Anzahl der in beschriebener Weise verknüpften Basiseinheiten ist in Figur 1b mit n bezeichnet.

Bei allen oben beschriebenen Ausführungsformen, die Benzolringe aufweisen (aromatische Polyether-Verbindungen, insbesondere PEEK, PEK, PEKK und PEI) können die Benzolringe einfach oder mehrfach substituiert sein. Die Substitution kann in meta- und/oder ortho-Stellung erfolgen. Die Substitution an einen Benzolring kann dabei an allen Benzolringen des gesamten Polymers identisch sein, sie kann aber auch verschieden sein.

Als Substituenten sowohl bei einfacher als auch bei mehrfacher Substitution können insbesondere die nachfolgend aufgeführten Substituenten verwendet werden: C1-C8-Alkyle, das heißt Methyl-, Ethyl-, Propyl-, Butyl-, Pentyl-, Hexyl-, Heptyl-, und/oder Octyl-Gruppen. Jedes der vorgenannten C1-C8-Alkyle kann dabei insbesondere gerade oder verzweigt sein. So sind ebenfalls iso-Propyl, sec-Butyl, tert-Butyl-Substituenten, sowie jegliche Isomere der C5-C8-Alkyl-Substituenten möglich. Des Weiteren kann es sich bei den Substituenten um C2-C8-Alkenyle handeln; das heißt um gerade oder verzweigte Ethenyl-, Propenyl-, Butenyl-, Pentenyl-,Hexenyl-, Heptenyl- und/oder Octenyl-Gruppen. Des Weiteren kann es sich bei den Substituenten um gerade oder verzweigte C2-C8-Alkinyl-Gruppen handeln; das heißt um Ethinyl-, Propinyl-, Butinyl-, Pentinyl-, Hexinyl-, Heptinyl- und/oder Oktinyl-Gruppen, die jeweils gerade oder verzweigt sind. Darüber hinaus kann es sich bei den Substituenten um C1-C8-Alkoxyl, das heißt Methoxy-, E-thoxy-, Propoxy-, Butoxy-, Pentoxy-, Hexoxy-, Heptoxy- und/ oder Oktoxy-Gruppen handeln.

Auch im Falle der Alkenyl- und/oder der Alkinylsubstituenten können jegliche denkbaren isomeren Formen substituiert sein.

Auch können die Substituenten cyclische, nichtaromatische oder aromatische C4-C8-Alkylsubstituenten, wie beispielsweise cyclo-Butyl-, cyclo-Pentyl-, cyclo-Hexyl-, cyclo-Heptyl- oder cyclo-Octyl-Reste sein.

Des Weiteren kann es sich bei den Substituenten um Chlor, Jod und/oder Brom handeln. Des Weiteren können die Substituenten aus einer Nitrogruppe, einer Hydroxylgruppe und/oder einer Aminogruppe bestehen bzw. diese Gruppen aufweisen. Es ist auch möglich, eine Phenyl-, Benzyl- und/oder Benzoyl-Gruppe als Substituenten zu verwenden.

Alle vorgenannten unsubstituierten, einfach oder mehrfach substituierten Polyether-Verbindungen sind erfindungsgemäß karbonfaserverstärkt. Zur Karbonfaserverstärkung können wahlweise Kurzfasern, Langfasern und/oder Endlosfasern verwendet werden. Als Kurzfasern werden dabei Fasern verstanden, die eine Länge zwischen 0,1 und 1 mm aufweisen. Als Langfasern werden Fasern bezeichnet, die eine Länge zwischen 1 und 50 mm aufweisen. Von Endlosfasern soll gesprochen werden, wenn ihre Länge 50 mm überschreitet. Die Endlosfasern können auf verschiedene Art und Weise verarbeitet worden sein, zum Beispiel in Form von Rovings, als Gewebe oder Gelege. Die Art der Faserverstärkung kann neben der Faserart auch durch den Volumenanteil der Faser, den diese insgesamt bei dem erfindungsgemäßen Befestigungsmittel einnimmt, beschrieben werden. Gemäß einer bevorzugten Ausführungsform weist wenigstens ein Teil des aus karbonfaserverstärktem unsubstituiertem, karbonfaserverstärktem einfachsubstituiertem oder karbonfaserverstärktem mehrfach substituiertem PEEK, PEK, PEKK oder PEI bestehenden Teiles eine Karbonfaserverstärkung mittels Langfasern oder Endlosfasern auf. Bevorzugt ist der Karbonfasergehalt des wenigstens einen Teiles, das mittels Langfasern oder Endlosfasern verstärkt ist, 50 bis 65 Volumenprozent, bevorzugt 55 bis 60 Volumenprozent (%vol).

Gemäß einer weiteren bevorzugten Ausführungsform weist wenigstens ein Teil des aus karbonfaserverstärktem unsubstituiertem, karbonfaserverstärktem einfach substituiertem oder karbonfaserverstärktem mehrfach substituiertem PEEK, PEK, PEKK oder PEI bestehenden Teiles eine Karbonfaserverstärkung mittels Kurzfasern auf. Bevorzugt liegt der Karbonfasergehalt des wenigstens einen Teiles, das mittels Kurzfasern verstärkt ist, zwischen 25 und 35 %vol., bevorzugt bei etwa 30 %vol.. Dabei sind fertigungsbedingte Toleranzen von +/- 2%vol., bevorzugt +/-1 %vol., höchstbevorzugt +/- 0,5%vol. zu berücksichtigen.

Wenigstens ein Teil des Befestigungsmittels soll aus den oben beschriebenen Materialien bestehen. Dies kann dadurch verwirklicht sein, dass das Befestigungsmittel selber mehrteilig ausgebildet ist und eines dieser Teile aus den beschriebenen Materialien aufgebaut ist bzw. besteht. Es ist auch möglich, dass das Befestigungsmittel selbst einteilig ausgebildet ist, wobei das einzelne Teil aus verschiedenen Materialien zusammengesetzt ist. Dies kann zum Beispiel durch einen Schichtaufbau erreicht werden, wobei eine Schicht aus den oben beschriebenen Materialien besteht.

Gemäß einer bevorzugten Ausführungsform weist ein erfindungsgemäßes Befestigungsmittel wenigstens ein weiteres Teil oder einen weiteren Teilbereich auf, der - anders als andere Teile der Erfindung - aus nicht-karbonfaserverstärktem unsubstituiertem, nicht-karbonfaserverstärktem einfachsubstituiertem oder nicht-karbonfaserverstärktem mehrfach substituiertem PEEK, PEK, PEKK oder PEI besteht. Dies kann zum Beispiel bei Teilen des Befestigungsmittels der Fall sein, die weniger starken Beanspruchungen ausgesetzt sind.

Gemäß einer bevorzugten Ausführungsform ist das Befestigungsmittel in Form einer Klemme ausgestaltet. Als Klemme wird dabei ein Gegenstand verstanden, der von wenigstens zwei Seiten einen Gegenstand zwischen diesen zwei Seiten fixieren kann, und zwar durch das Aufbringen von Haltekräften. Dabei ist es möglich, dass in den zu haltenden Körper durch die Klemme eingedrungen wird, es ist aber auch möglich, dass der Körper unversehrt bleibt.

Gemäß einer bevorzugten Ausführungsform ist die Klemme an einem Knochen spannungsbalanciert anbringbar. Darunter ist folgendes zu verstehen: Wird eine Klemme an einem Knochen angebracht, so treten dabei neben den normalen Haltekräften auch Biegekräfte auf. Die gezielte Vermeidung dieser Biegekräfte innerhalb von Teilen, bei denen sich diese Art der Belastung negativ bemerkbar machen würde, ist Grundlage für die spannungsbalancierte Ausführungsform. Auf Teile der Klemme wirken Kräfte in symmetrischer Art und Weise ein, das heißt, in gleicher Stärke und in symmetrischer Art und Weise hinsichtlich der Richtungen der Kräfte, so dass sich diese gegenseitig kompensieren. Dies wird bei herkömmlichen Klemmen nicht berücksichtigt. Dadurch kann es zu Schwergängikeit des Befestigungsmechanismus, mechanischen Verformungen und Auswirkungen auf die Genauigkeiten der Referenzgeometrienabringung kommen, oder es hätte zur Folge, dass die Konstruktion größer dimensioniert werden müsste, was für den vorgesehen Verwendungszweck nicht akzeptabel wäre.

Gemäß einer bevorzugten Ausführungsform weist die Klemme einen Klemmbereich und einen Trägerbereich mit einem Übertragungsabschnitt auf, wobei der Klemmbereich und der Übertragungsabschnitt miteinander in Kontakt stehen. Der Klemmbereich ist angepasst, um die Klemme an einem Knochen zu befestigen. Eine Wechselwirkung zwischen dem Übertragungsabschnitt und dem Klemmbereich erfolgt bei einem Klemmvorgang spannungsbalanciert. Zum Klemmbereich der Klemme werden all diejenigen Bestandteile der Klemme gezählt, die für den Fixierungsvorgang bzw. Klemmvorgang wesentlich sind. Es sind dies insbesondere die Teile, die zum Festklemmen in Bewegung versetzt werden. Zum Trägerbereich der Klemme zählt letztlich alles, was nicht zum Klemmbereich gehört. Der Teil des Trägerbereiches, der mit dem Klemmbereich in Kontakt steht, ist wegen der dort auftretenden Wechselwirkung als Übertragungsabschnitt bezeichnet. Auf den Klemmbereich wirken Biegekräfte bei der Befestigung ein. Im Klemmbereich kommt es insofern auch zu auftretenden Verspannungen. Ebenso verhält es sich am Übcrtragungsabschnitt des Trägerbereiches. Innerhalb des Übertragungsabschnittes werden allerdings nur Kräfte in symmetrischer Art und Weise eingeleitet und dadurch kompensiert, so dass keinerlei Biegebelastungen an den Trägerbereich übertragen werden. Gemäß einer bevorzugten Ausführungsform weist der Trägerbereich des Weiteren einen Lagerabschnitt auf. Der Klemmbereich weist zwei Schenkel auf, die jeweils drehbar um eine Achse gelagert sind, die jeweils durch den Lagerabschnitt hindurchgeführt ist. Die Klemme ist so ausgebildet, dass der Übertragungsabschnitt mit beiden Schenkeln zugleich symmetrisch in Wechselwirkung tritt und dadurch eine Rotation der Schenkel um ihre Rotationsachsen vom gleichen Betrage, aber in entgegengesetzter Drehrichtung bewirkt, so dass die Klemme am Knochen spannungsbalanciert anbringbar ist. Bevorzugt liegen die beiden Rotationsachsen in derselben Ebene in jeweils gleichem Abstand von den Endpunkten des jeweiligen Schenkels des Klemmbereiches.

Gemäß einer weiteren bevorzugten Ausführungsform weist der Lagerabschnitt eine Hauptachse auf, der Übertragungsabschnitt ist in einer sich verjüngenden Form ausgebildet, und das Befestigungsmittel ist so ausgebildet, dass der sich verjüngende Übertragungsabschnitt entlang der Hauptachse des Lagerabschnittes bewegt und dabei die Schenkel des Klemmbereiches in Rotation versetzt. Der sich verjüngende Übertragungsabschnitt kann zum Beispiel eine Keilform aufweisen oder in der Form eines Kegelstumpfes ausgebildet sein. Unter einer Keilform wird dabei eine dreidimensionale Form verstanden, die sich wenigstens in einer Dimension von einem Ende zum anderen Ende hin verjüngt. Diese Verjüngung kann aber auch in zwei Dimensionen erfolgen. Unter der Hauptachse ist streng genommen die Hauptachse mit dem geringsten Trägheitsmoment zu verstehen, also im Prinzip das, was im Umgangssprachgebrauch gemeinhin als Achse bezeichnet wird, weil es die größte Längsausdehnung aufweist. Bei der Bewegung des keilförmigen Übertragungsabschnittes entlang der Hauptachse des Lagerabschnittes kann es sich um einen Gleitvorgang handeln. Dieser kann allein durch eine translatorische Bewegung des keilförmigen Übertragungsabschnittes bezogen auf die Schenkel des Klemmbereiches erfolgen. Es ist aber auch möglich, dass der Übertragungsabschnitt nicht keilförmig, sondern zum Beispiel kegelstumpfförmig ausgebildet ist, wobei sich die Relativbewegung dann nicht in einer translatorischen Bewegung erschöpfen müsste, sondern zusätzlich auch eine Drehbewegung möglich wäre.

Gemäß einer bevorzugten Ausführungsform ist der Übertragungsabschnitt keilförmig ausgebildet und weist eine Führung für jeweils ein Ende der Schenkel auf. Dabei können zum Beispiel Vorsprünge der Schenkel in der Führung, die am keilförmigen Übertragungsabschnitt angebracht ist, entlang gleiten. Bei der Führung kann es sich um die Positivform oder die Negativform einer Führung handeln, das heißt die Schenkel können mit ihren Enden zum Beispiel in die Führung eingreifen und hineingesteckt sein, sie können diese aber auch von außen in geeigneter Weise umschließen, um so geführt zu werden.

Gemäß einer bevorzugten Ausführungsform weist der Trägerbereich einen Betätigungsabschnitt auf, der bei Betätigung den Übertragungsabschnitt in Bewegung versetzt. Dabei kann es sich zum Beispiel um ein Schraubelement handeln, das bei entsprechender Drehbewegung sich entlang einer Schraube in deren axialer Richtung bewegt und auf den Übertragungsabschnitt eine Kraft ausübt, die den Übertragungsabschnitt zum Beispiel in eine translatorische Bewegung und/oder eine Rotationsbewegung versetzt.

Gemäß einer bevorzugten Ausführungsform weist ein erfindungsgemäßes Befestigungsmittel wenigstens einen Eindringkörper auf, der aus einer Siliziumnitrid-Keramik bevorzugt einer β-Siliziumnitrid-Keramik, besteht. Unter einem Eindringkörper wird ein Keil oder ein Bereich verstanden, der bei der Fixierung in den Körper, an dem die Fixierung erfolgen soll, eindringt. Im Falle von Knochen eignen sich hierfür insbesondere sogenannte Pins bzw. Spikes, die mit einer Spitze versehen sind und starken Belastungen standhalten können. Bevorzugt sind mehrere Eindringkörper vorgesehen, bevorzugt befinden sich diese im Falle einer Klemme als Befestigungsmittel auf beiden Schenkeln der Klemme. Bei der Siliziumnitrid-Keramik handelt es sich bevorzugt um eine β-Siliziumnitrid-Keramik. Es ist möglich, dass der Siliziumnitrid-Keramik bzw. β-Siliziumnitrid-Keramik andere Keramiken beigemischt bzw. beigefügt sind.

Gemäß einer bevorzugten Ausführungsform handelt es sich um ein Befestigungsmittel mit wenigstens einem Eindringkörper, der an dem Befestigungsmittel mittels einer Verliersicherung angebracht ist. Eine solche Verliersicherung ist ein Bestandteil des Befestigungsmittels, das verhindert, dass beim Lösen des Befestigungsmittels Eindringkörper in dem Körper, an dem die Fixierung vorgenommen wurde, zum Beispiel einem Knochen, verbleiben. Es wird auch verhindert, dass sich die Eindringkörper, nachdem sie bereits locker geworden sind, zwar entfernen lassen, jedoch dann beim Gesamtentfernungsvorgang herabfallen und in eine offene Wunde gelangen und dort möglicherweise nicht mehr auffindbar sind.

Gemäß einer bevorzugten Ausführungsform weist die Verliersicherung ein Plättchen auf, durch das der wenigstens eine Eindringkörper teilweise hindurchragt, wobei das Plättchen einen Teil des Eindringkörpers, der nicht durch das Plättchen hindurchragt, zwischen dem Befestigungsmittel und dem Plättchen einklemmt. Dies kann typischerweise dadurch erreicht werden, dass die Eindringkörper (von der Spitze einmal abgesehen) nicht überall denselben Durchmesser aufweisen. Sie können sich zum Beispiel weg von der Spitze stufenweise oder kontinuierlich verbreitern, so dass sie nicht komplett durch das Plättchen hindurchpassen. Bevorzugt ist eine stufenweise Verbreiterung des Durchmessers und eine dadurch mögliche flache Auflageweise des Plättchens über einen Teil des Eindringkörpers.

Gemäß einer bevorzugten Ausführungsform ist die Verliersicherung mittels eines Klickverschlusses an dem Befestigungsmittel befestigbar. Dies kann zum Beispiel durch Einrasten von kleinen Vorsprüngen gewährleistet sein. Alternativ ist auch denkbar, dass die Verliersicherung angeschraubt oder angeklebt wird.

Gemäß einem weiteren Aspekt der Erfindung bezieht sich diese auf ein System umfassend ein Befestigungsmittel, wie es oben in den unterschiedlichsten Ausführungsformen beschrieben wurde, und eine Referenzgeometrie für navigationsunterstützte Operationen. Diese kann an dem Befestigungsmittel befestigt sein, oder aber lediglich daran befestigbar sein. Bei der Referenzgeometrie handelt es sich um die an sich bekannten Markereinrichtungen, mit aktiven oder passiven Markern, wobei sich die Marker zueinander in einer definierten bekannten Position befinden und von einem Kamerasystem aufgrund der von ihnen ausgesandten Strahlung oder aufgrund der von ihnen reflektierten Strahlung erfasst werden können.

Die Erfindung wird noch besser verstanden werden unter Bezugnahme auf die beiliegenden Zeichnungen. Dabei zeigen:
- Figur 1a, 1b: zeigen die Benennungssystematik von Polyaryletherketonen (PEK, PEEK und PEKK) und von Polyetherimiden (PEI);
- Figur 2: zeigt herkömmliche Klemmen aus dem Stand der Technik mit einer Referenzgeometrie;
- Figuren 3a, 3b: zeigen Röntgenschnittaufnahmen mit starken Artefakten aufgenommen mit einem Computertomographen, die bei Fixierung einer Klemme aus Edelstahl aufgenommen worden sind;
- Figuren 4a, 4b: zeigen Röntgenschnittaufnahmen mit geringen Artefakten aufgenommen mit einem Computertomographen, die bei Fixierung einer erfindungsgemäßen Klemme aufgenommen worden sind;
- Figur 5: zeigt eine Röntgenschnittaufnahme mit Artefakten aufgenommen mit einem Computertomographen, die durch die Verwendung einer herkömmlichen Referenzgeometrie entstanden sind;
- Figuren 6a, 6b: zeigen eine dorsale und laterale 3D-Rekonstruktion eines Röntgenbildes, wobei zum Vergleich sowohl eine Klemme aus Edelstahl als auch eine erfindungsgemäße Klemme aus kohlenfaserverstärktem PEEK mit abgebildet wurden, die unterschiedliche Arten von Artefakten verursachen;
- Figuren 7a, 7b: zeigen das Artefaktverhalten auf genommen mit einem Computertomographen bei Verwendung eines Titaniumpins bzw. bei Verwendung eines Siliziumnitrid-Keramik-Pins;
- Figur 8: zeigt eine erfindungsgemäße Klemme, die am Dornfortsatz eines Wirbelkörpers befestigt ist;
- Figur 9: zeigt eine erfindungsgemäße Klemme im Detail;
- Figur 10: zeigt eine Explosionsansicht der Klemme, die in Figur 10 gezeigt ist;
- Figur 11: zeigt ein Befestigungsmittel mit einer Schraube;
- Figur 12: zeigt ein Befestigungsmittel mit zwei Schrauben.

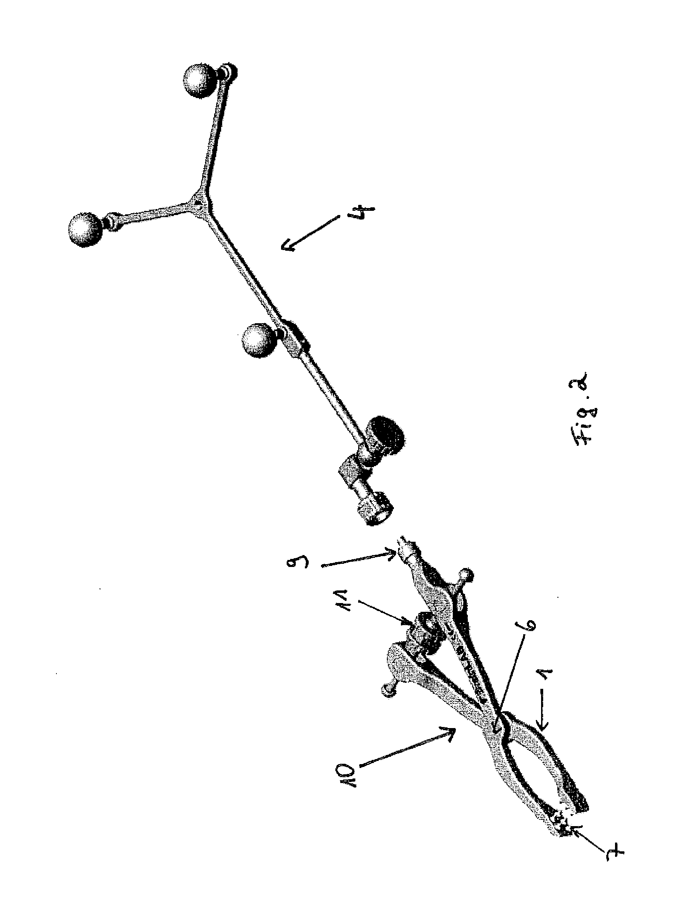

Figur 2 zeigt eine Klemme 10, die mit einer Referenzgeometrie 4 ausgestattet werden kann, die gemeinhin aus Edelstahl hergestellt ist. Die herkömmliche Klemme gemäß dem Stand der Technik ist nicht strahlendurchlässig. Sie weist zwei Klemmbacken bzw. Schenkel 1 auf, die um eine gemeinsame Achse 6 drehbar gelagert sind. An ihrem einen Ende sind die Schenkel 1 mit Spikes 7 ausgestattet, die sich in einen Knochen hineinbohren können. Am entgegengesetzten Ende der Schenkel ist eine Verschraubung bzw. Schraubverbindung 11 zwischen den beiden Schenkeln 1 vorgesehen. Durch Verlängern oder Verkürzen des Abstandes zwischen den beiden Spike-fernen Enden der Schenkel 1 wird die Klemme 10 geöffnet oder geschlossen und festgezogen. Die Klemme 10 hat eine Schnittstelle 9 an die eine Referenzgeometrie 4 befestigt werden kann.

Alle Komponenten, die in Figur 2 gezeigt sind (Klemme 10 und Referenzgeometrie 4) bestehen aus Edelstahl und sind nicht strahlendurchlässig. Anders verhält es sich mit einer erfindungsgemäßen Klemme, die wenigstens teilweise aus einer karbonfaserverstärkten unsubstituierten, karbonfaserverstärken einfachnsubstituierten oder karbonfaserverstärkten mehrfach substituierten Polyether-Verbindung besteht. Alle genannten Polyether-Verbindungs-Varianten sind strahlendurchlässig.

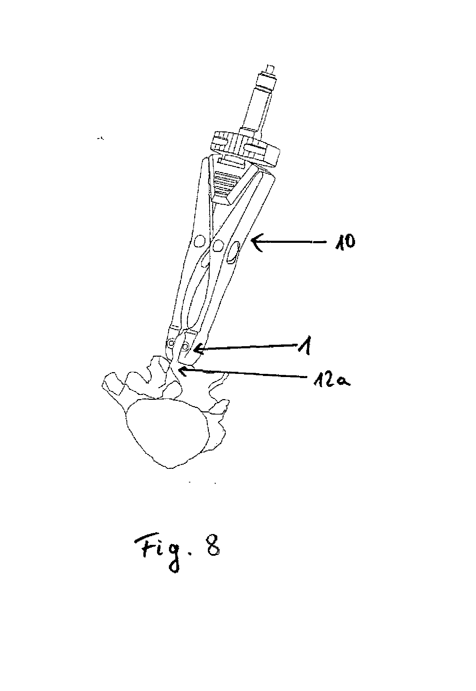

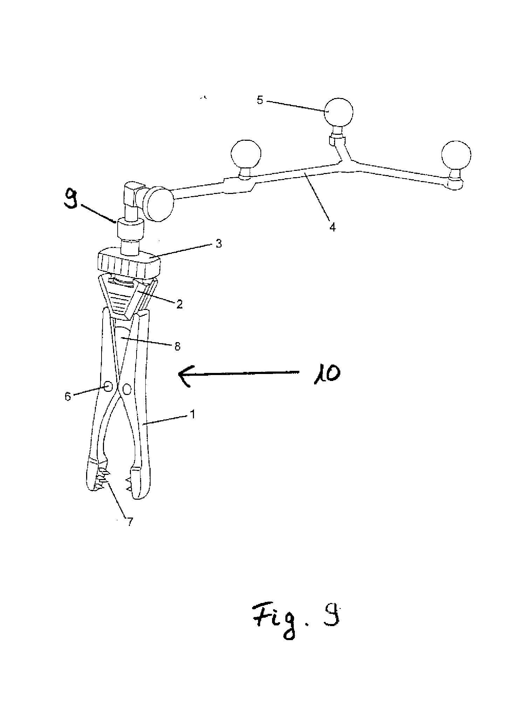

Figur 8 zeigt die Geometrie eines erfindungsgemäßen Befestigungsmittels, das in Form einer Klemme 10 ausgebildet ist. Die Klemme 10 ist mittels zweier Klemmbacken 1 am Dornfortsatz eines Wirbelkörpers 12a befestigt. Figur 9 zeigt die Klemme 10 mit einer Referenzgeometrie 4, die daran über eine Schnittstelle 9 befestigt ist, im Detail. Die Referenzgeometrie 4 war im Falle aller Testaufnahmen aus Edelstahl hergestellt. Es ist aber auch denkbar, dass für die Referenzgeometrie 4 ein strahlendurchlässiges Material wie zum Beispiel Kunststoff oder eine bestimmte Keramik verwendet wird. Die Klemme in Figur 9 weist zwei einander gegenüberliegender Schenkel 1 in Form von Klemmbacken auf. An den Enden, an denen ein Kontakt zum Knochen hergestellt werden soll, weisen die Schenkel 1 jeweils Spikes 7 auf. Diese sind aus Siliziumnitrid-Keramik hergestellt (SNI 750; vgl. Datenblatt 4 im Anhang). Die Klemme 10 weist des Weiteren einen Hauptstab 8 auf, der zwei Rotationsachsen 6 beinhaltet, in denen die Schenkel 1 jeweils gelagert sind. Ein Keilstück 2, das als Übertragungsabschnitt dient, ist in Kontakt mit einem Drehrad 3, welches eine Schraubverbindung zu dem Hauptstab 8 aufweist. Durch Drehen des Drehrades 3 bewegt sich das Keilstück 2 abwärts, weshalb sich dann die Klemmbacken 1 schließen. Die Klemmkraft wird auf das Keilstück 2 symmetrisch übertragen, aus diesem Grunde tritt keine kritische Biegekraft am Hauptstab 8 auf, die zu einer Verschiebung der Referenz führen könnte. Die Schenkel 1 weisen Elemente auf, die in entsprechende Führungsschlitze am Keilstück 2 eingreifen und auf diese Weise mit dem Keilstück 2 verbunden sind, und die dafür sorgen, dass sich die Klemmbacken automatisch öffnen, wenn das Drehrad 3 in die entgegengesetzte Richtung gedreht wird. Das Drehrad 3 kann einfach von dem Keilstück 2 für Reinigungs- und Sterilisationszwecke entfernt werden. Eine Schnittstelle 9 sorgt dafür, dass die Referenzgeometrie 4 an und abgebaut werden kann. Die Referenzgeometrie 4 weist im dargestellten Fall mindestens drei Marker 5 auf, die sich in einem bekannten definierten Abstand zueinander befinden.

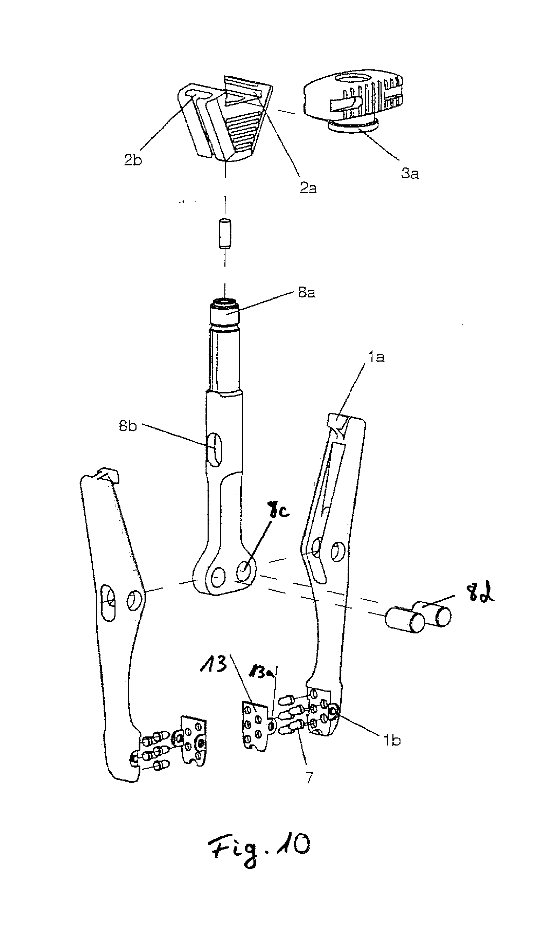

In Figur 10 ist die Klemme 10 in einer Explosionsdarstellung in größerer Detailtiefe dargestellt. Zunächst ist der Aufbau des Hauptstabes 8 im Detail zu erkennen. Der Hauptstab 8 weist im oberen Bereich (oben und unten jeweils bezogen auf die Position in der Abbildung) einen zylinderförmigen Abschnitt 8a auf, der die Schnittstelle für die Referenzgeometrie beinhaltet. In der Mitte weist der Hauptstab 8 eine Aussparung 8b auf, die im vorliegenden Fall ovalförmig ausgebildet ist. In diese Aussparung 8b können Häkchen 1a der Schenkel 1 eingreifen. Dies ermöglicht eine besonders platzsparende Ausführung der Klemme, die eine bessere minimal invasive Anwendung der erfindungsgemäßen Klemme ermöglicht. Im unteren Bereich weist der Hauptstab 8 zwei Öffnungen 8c auf, in die Achsen 8d aufgenommen und gleichzeitig durch die Schenkel 1 durchgeführt werden können, so dass die Rotationsachsen für beide Schenkel 1 gebildet werden können.

Beim Keilstück 2, das als Übertragungsabschnitt dient, ist eine Aussparung 2b zu erkennen, die als Führung für die Schenkel 1 dient, die mittels der Häkchen 1a in die Führung 2b eingreifen. Das Drehrad 3 wird an dem Keilstück dadurch fixiert, dass ein Kragen 3a in eine entsprechend ausgeführte Aussparung 2a des Keilstückes 2 hineinschiebbar ist. Das Drehrad 3, das als Betätigungsstück dient, kann auf das Keilstück aufgesetzt mit dem Hauptstab 8 verschraubt werden. Wird das Drehrad 3 betätigt, das heißt gedreht, wird durch den Kontakt zum Keilstück 2 dieses entlang des Hauptstabes 8 bewegt bzw. hinuntergeschoben, und die Schenkel 1 werden gespreizt. Dabei rutschen die Häkchen 1a der Schenkel 1 in der Führung 2b des Keilstückes 2 in dem Keilstück 2 nach oben, das heißt in einen Bereich, der verbreitert ist. Das Keilstück 2 ist spiegelsymmetrisch bezüglich einer gedachten Achse A ausgebildet. Aus diesem Grunde werden die Schenkel 1 bzw. die Häkchen 1a symmetrisch bewegt. Die von dem Keilstück 2 auf die Schenkel 1 ausgeübte Kraft ist deshalb ebenfalls symmetrisch, weshalb keine kritische Biegekräfte am Hauptstab 8 auftreten.

Im Klemmbereich der Klemme (10) sind mehrere Spikes 7 zu erkennen. Diese sind in die Klemmbacken 1 hineingepresst oder hineingeklebt. Es ist zu erkennen, dass die Spikes 7 an dem Ende, das ihrer Spitze entgegengesetzt liegt, eine leichte Verdickung aufweisen, das heißt der Durchmesser der Spikes (von der Spitze einmal abgesehen) variiert. Ein Plättchen 13, das mit Löchern ausgestattet ist, wird nun über die Spikes 7 geschoben, wobei nur die mit geringerem Durchmesser ausgestatteten Bereiche der Spikes durch die Löcher des Plättchens 13 hindurchpassen. Der verdickte Bereich der Spikes 7 wird zwischen dem Plättchen 13 und der Klemmbacke 1 eingeklemmt. Das Plättchen 13 dient somit als Verliersicherung. Es lässt sich mittels eines Klickverschlusses 1b an der Klemmbacke befestigen.

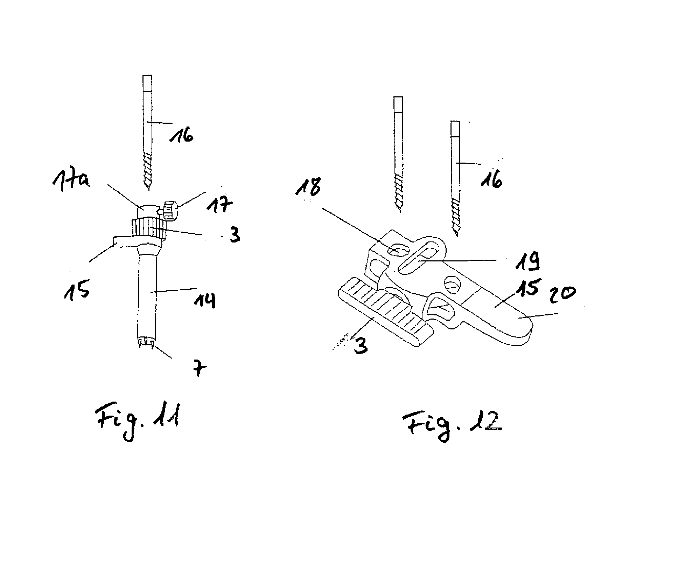

In den Figuren 11 und 12 sind zwei weitere Befestigungsmittel dargestellt, die nicht in Form einer Klemme ausgebildet sind. Stattdessen wird in Figur 11 ein Befestigungsmittel mit einer Schraube 16 illustriert, in Figur 12 wird ein Befestigungsmittel mit zwei Schrauben 16 dargestellt. Die dargestellten Befestigungsmittel sind hinsichtlich ihrer Geometrien bekannt, sie sind aber im vorliegenden Fall wenigstens teilweise aus karbonfaserverstärktem unsubstituiertem, karbonfaserverstärktem einfach substituiertem oder karbonfaserverstärktem mehrfach substituiertem PEEK hergestellt. Dabei können wie im allgemeinen Teil der Beschreibung der Erfindung beschrieben verschiedenste Fasertypen und Karbonfasergehalte in Volumenprozent verwendet werden.

In Figur 11 weist das Befestigungsmittel ein Hauptstück 14 auf, an das mittels einer Schnittstelle 15 eine Referenzgeometrie angebracht werden kann. Das Hauptstück 14 wird über eine Knochenschraube 16, die in den Knochen eingeschraubt ist, angebracht. Ein Verschlussring 17a wird mittels eines Klemmmechanismus 17 gegen die Schraube 16 gedrückt, wodurch es zu einer Fixierung kommt. Schließlich wird das Rädchen 3 gedreht, und es wird eine Kraft auf das Hauptstück 14 ausgeübt. Dies drückt die Spikes 7 in die Knochenstruktur hinein. Durch Anwenden dieses Verfahrens kann eine feste Verbindung mit der Knochenstruktur erreicht werden. Die Spikes 7 können entweder aus Kunststoff oder aus Keramikmaterialien gefertigt sein.

Bei der Fixierung in Figur 12 werden zwei Knochenschrauben 16 verwendet, die mit der Knochenstruktur in einer bestimmten Distanz zueinander befestigt werden. Eine Bohrschablone kann diesen Vorgang verbessern. Das Hauptstück 20 wird mit den Öffnungen 18 auf zwei Knochenschrauben 16, die in den Knochen eingeschraubt sind, gesteckt. Das Hauptstück 20 hat eine Schnittstelle an die eine Referenzgeometrie angebracht werden kann. Das Rad 3 bewegt ein keilförmiges Teilstück 19 im Inneren des Hauptstückes 20 wodurch das Hauptstück 20 fest mit den zwei Schrauben 16 verklemmt wird. An einer Schnittstelle 15 des Hauptstückes 20 kann wiederum eine Referenzgeometrie angebracht werden.

Es ist nicht möglich, Befestigungsmittel, insbesondere Klemmen, gemäß dem Stand der Technik, die bevorzugt aus Titan oder Edelstahl hergestellt sind, einfach aus einem anderen Material zu fertigen, ohne dabei weitere Anpassungen der Befestigungsmittel vorzunehmen. Insbesondere ist es nicht möglich, die in Figur 2 gezeigten Klemmen statt aus Edelstahl aus nichtkarbonfaserverstärktem PEEK - gleich in welcher Substitution - herzustellen. In diesem Fall würde die Klemme aus nicht-karbonfaserverstärktem PEEK eine Größe aufweisen müssen, die um den Faktor 2 bis 3 größer ist, als die der bisher gemäß dem Stand der Technik verwendete Edelstahlklemme. Andernfalls würde nicht die notwendige Festigkeit des Befestigungsmittels erreicht. Selbstverständlich ist eine solche Größensteigerung für ein Befestigungsmittel, das bei navigationsunterstützten Operationen eingesetzt wird, nicht aktzeptabel, da dadurch Bewegungsfreiheit und Sicht des Operateurs zu stark behindert würde und eine mögliche minimal-invasive Anwendung nicht mehr gegeben wäre.

Mit einem Prototyp eines erfindungsgemäßen Befestigungsmittels wurden mehrere Versuchsreihen durchgeführt, um das Artefaktverhalten der erfindungsgemäßen Befestigungsmittel in verschiedenen Aufnahmen, insbesondere in 2D-Röntgenschnittaufnahmen und bei 3D-Röntgenaufnahmen, die aus 2D-Bildern zusammengesetzt und rekonstruiert sind, zu untersuchen. Dabei wurde der Klemmen-Prototyp, dessen geometrische Erscheinungsform in den Figuren 9 und 10 dargestellt ist, verwendet. Er bestand aus unterschiedlichen PEEK-Materialien: die Schenkel 1 und die Achsen 8d bestanden aus karbonfaserverstärktem unsubstituiertem PEEK, wobei die Verstärkung mittels Endlosfasern erfolgte, und wobei der Karbonfasergehalt 55 bis 60 Volumenprozent betrug (vgl. anhängendes Datenblatt 1). Der Hauptstab 8 und das Keilstück 3 bestanden aus karbonfaserverstärktem unsubstituiertem PEEK, wobei die Verstärkung mittels Kurzfasern erfolgte und der Karbonfasergehalt etwa 30 Volumenprozent betrug (vgl. Datenblatt 2 im Anhang). Das Plättchen 13 und das Drehrad 3 bestanden aus nichtkarbonfaserverstärktem unsubstituiertem PEEK (vgl. Datenblatt 3 im Anhang). Die Spikes 7 bestanden aus Siliziumnitrid-Keramik (vgl. Datenblatt 4 im Anhang). Dass das Prototyp-Befestigungsmittel in Form einer Klemme aus unterschiedlichen PEEK-Materialien besteht, ist aus folgenden Gründen möglich: Die auf das Befestigungsmittel einwirkenden Kräfte unterscheiden sich hinsichtlich der einzelnen Bestandteile des Befestigungsmittels. Während auf die Achsen 8d und die Schenkel 1 hohe Biegekräfte einwirken können, ist dies beim Hauptstab 8 und Keilstück 2 nicht der Fall, ganz zu schweigen vom Drehrad 3. Es macht deshalb Sinn, unterschiedliche PEEK-Materialien zu verwenden, wobei das Material mit der höchsten Beanspruchbarkeit und Biegefestigkeit für die am meisten beanspruchten Teile ausgewählt wird. Damit in Zusammenhang stehend ist erforderlich, dass die Kraftübertragung zwischen Klemmbereich und Trägerbereich spannungsbalanciert erfolgt, da ansonsten nicht hinsichtlich der Beanspruchbarkeit der unterschiedlichen Teile der Klemme 10 in genannter Weise unterschieden werden könnte. Die Prototyp-Klemme 10 kann einfach zerlegt, gereinigt bzw. sterilisiert und wieder zusammengesetzt werden. Auch dies macht es möglich, unterschiedliche Materialien für unterschiedliche Bestandteile auszuwählen. Bei der Materialauswahl der Spikes 7 wurde beachtet, dass es einerseits galt, ein besonders festes und mechanisch beanspruchbares Material auszuwählen, dass andererseits aber nur geringe Artefakte in 2D-Röntgenschnittaufnahmen und rekonstruierten 3D Röntgenbildern verursacht. Siliziumnitrid-Keramik erwies sich dabei als der ideale Kandidat.

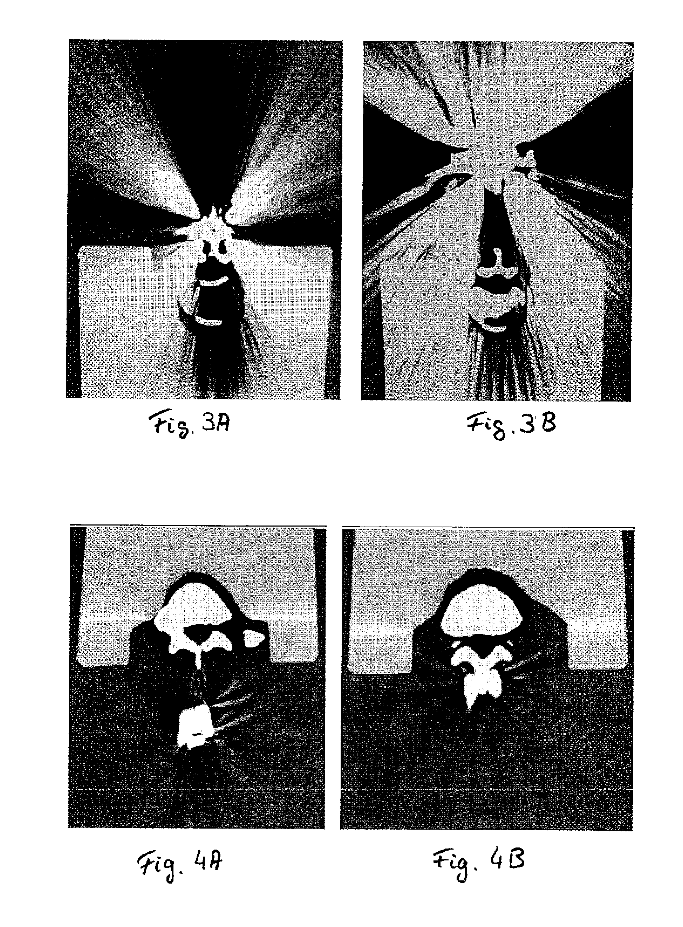

Die Figuren 3a und 3b zeigen einzelne Röntgenschnittaufnahmen aus einem rekonstruiertem 3D Röntgenmodell, das mit einem Computertomographen aufgenommen wurde. In beiden Fällen wurde ein Schnitt durch einen bestimmten Wirbel der Wirbelsäule aufgenommen, wobei eine herkömmliche Klemme aus Edelstahl an einem Wirbelkörper fixiert war. Es ist in den Figuren 3a und 3b eine starke Artefaktbildung zu erkennen. Das Bild ist von starken sternförmig angeordneten Linien in schwarz und weiß überlagert, so dass die weißen Wirbelkörper im Bildzentrum kaum noch zu erkennen sind. Die Bilder sind praktisch für navigationsgestütze Operationen ungeeignet, da relevante Knochenstrukturen am Wirbelkörper, in welche mittels Navigationsunterstützung zum Beispiel Schrauben eingebracht werden müssen, nicht mehr erkannt werden.

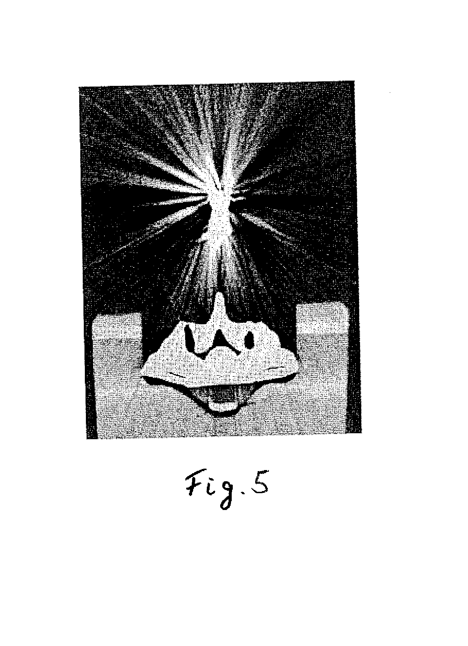

Figuren 4a und 4b zeigen zum Vergleich zwei unterschiedliche 2D-Schnitte durch einen Wirbelkörper aus einem rekonstruiertem 3D Röntgenmodell, das mit einem Computertomographen aufgenommen wurde, wobei eine erfindungsgemäße strahlendurchlässige Klemme aus karbonfaserverstärktem PEEK verwendet wurde. Außerdem war die Klemme aus karbonfaserverstärktem PEEK mit Spikes aus einer Siliziumnitrid-Keramik (SNI 750 gemäß Datenblatt 4) versehen. Die Spikes aus Siliziumnitrid-Keramik halten verhältnismäßig starken mechanischen Belastungen stand. Die leichten Artefakte, die in den Figuren 5a und 5b zu erkennen sind, resultieren aus der Verwendung der Silizimnnitrid-Keramik-Pins. Dennoch ist festzuhalten, dass diese Artefakte in ihrer Ausprägung so schwach sind, dass sie die Bildqualität nicht merklich negativ beeinflussen.

In Figur 5 ist eine Schnittaufnahme durch einen Wirbelkörper aus einem rekonstruiertem 3D Röntgenmodell gezeigt, das mit einem Computertomographen aufgenommen wurde, wobei eine erfindungsgemäße Klemme aus karbonfaserverstärkten PEEK verwendet wurde, an der eine herkömmliche Referenzgeometrie bestehend aus Edelstahl befestigt war. Diese befindet sich in einem gewissen Abstand zum Wirbelkörper, an dem die Klemme fixiert ist. Die durch die Verwendung der bekannten Referenzgeometrie aus Edelstahl hervorgerufenen Artefakte sind zwar stark, befinden sich aber in einem gewissen Abstand zum Wirbelkörper, der von Interesse ist. Es ist festzuhalten, dass dieser Abstand groß genug ist, so dass auch bei der Verwendung von Refcrenzgeometrien des Standes der Technik aus Edelstahl in Kombination mit der erfindungsgemäßen Klemme aus karbonfaserverstärkten PEEK gute rekonstruierte 2D-Röntgenschnittaufnahmen erhalten werden können.

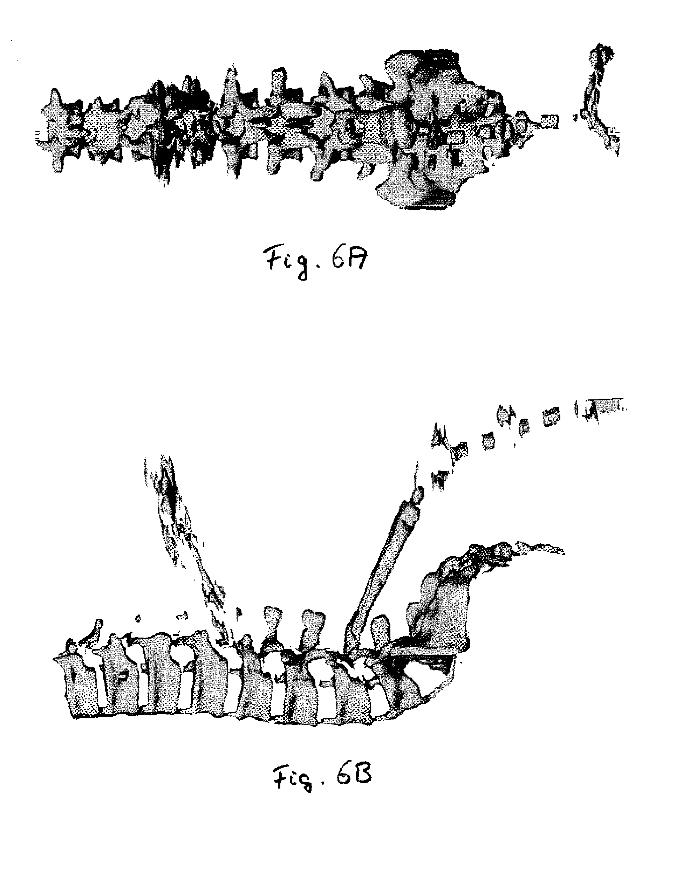

Die Figuren 6a und 6b zeigen 3D-Rekonstruktionen, die mit einem Computertomographen aufgenommen wurden, wobei Figur 6a eine dorsale 3D-Rekonstruktion und Figur 6b eine laterale 3D-Rekonstruktion zeigt. Wiederum war im linken Bereich eine Klemme aus Edelstahl angebracht, während im rechten Bereich der erfindungsgemäße Prototyp befestigt war. Es ist zu erkennen, dass links im Bereich der Edelstahlklemme wesentlich stärkere Artefakte zu verzeichnen sind und sogar relevante Bereiche der Wirbelsäule wie z.B. Dornfortsätze komplett fehlen, während hingegen im rechten Bereich die vollständige Kontur der Klemme zu erkennen ist und darunter liegende Bereich der Wirbelsäule vollständig rekonstruiert sind. Dies gilt insbesondere für die dorsale 3D-Rekonstruktion.

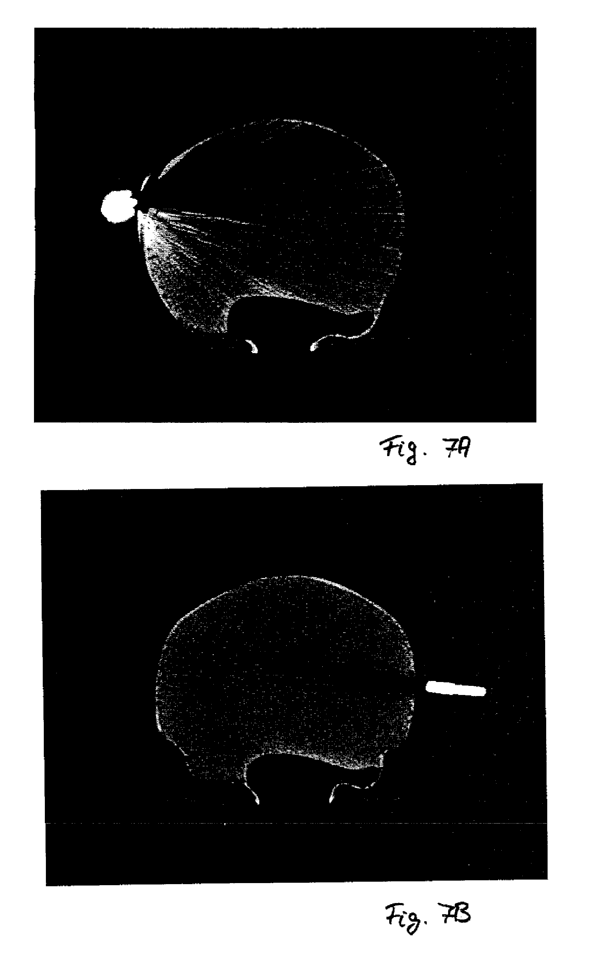

Die Figuren 7a und 7b zeigen das unterschiedliche Artefaktverhalten bei der Verwendung eines Titanium-Pins und der Verwendung eines Siliziumnitrid-Keramik-Pins bei einer 3D-rekonstruierten Röntgenschnittaufnahme. Hierzu wurden stabförmige Testkörper aus den genannten Materialien verwendet. Bei der Verwendung eines Titanium-Pins treten starke Artefakte auf, während bei der Verwendung des Siliziumnitrid-Keramik-Pins nur schwache und gerichtete Artefakte auftreten.

Aus allen Aufnahmen, die hinsichtlich ihrer Qualität miteinander verglichen wurden, geht hervor, dass aufgrund der erfindungsgemäßen Materialauswahl wesentlich bessere Ergebnisse hinsichtlich des Artefaktverhaltens in Röntgenaufnahmen erhalten wurden. Durch Verwendung einer Klemme aus karbonfaserverstärktem unsubstituiertem, karbonfaserverstärktem einfachsubstituiertem oder karbonfaserverstärktem mehrfach substituiertem PEEK wurde es möglich, Röntgenaufnahmen zu tätigen und 3D Bildrekonstruktionen zu erhalten, die nur sehr geringe Artefakte aufweisen und deshalb eine große Anzahl von Details sehr gut erkennen lassen, was hinsichtlich navigationsgestützen Operationen, die mit höchster Präzision durchgeführt werden müssen, von großem Vorteil ist. Dabei wurde durch die Verwendung der beschriebenen karbonfaserverstärkten-PEEK-Materialien gleichzeitig eine hohe mechanische Beanspruchbarkeit des Befestigungsmittels gewährleistet und der erforderliche Miniaturisierungsgrad des Befestigungsmittels, insbesondere der Klemme, erreicht, wodurch eine minimal-invasive Anwendung möglich ist.

### Bezugszeichenliste

- 1: Klemmbacke (Schenkel)
- 1a: Häkchen
- 1b: Klickverschluss
- 2: Keilstück (Übertragungsabschnitt)
- 2a: Aussparung des Keilstückes
- 2b: Aussparung / Führung
- 3: Drehrad
- 3a: Kragen
- 4: Referenzgeometrie
- 5: Marker
- 6: Rotationsachse
- 7: Spike
- 8: Hauptstab
- 8a: zylinderförmiger Abschnitt mit Gewinde
- 8b: Aussparung
- 8c: Öffnung
- 8d: Stäbchen
- 9: Schnittstelle
- 10: Klemme
- 11: Schraubverbindung
- 12: Wirbelsäule
- 12a: Wirbelkörper
- 13: Verliersicherung
- 13a: Öffnung
- 14: Hauptstück
- 15: Schnittstelle
- 16: Knochenschraube
- 17: Klemmmechanismus
- 18: Loch
- 19: Keilstück

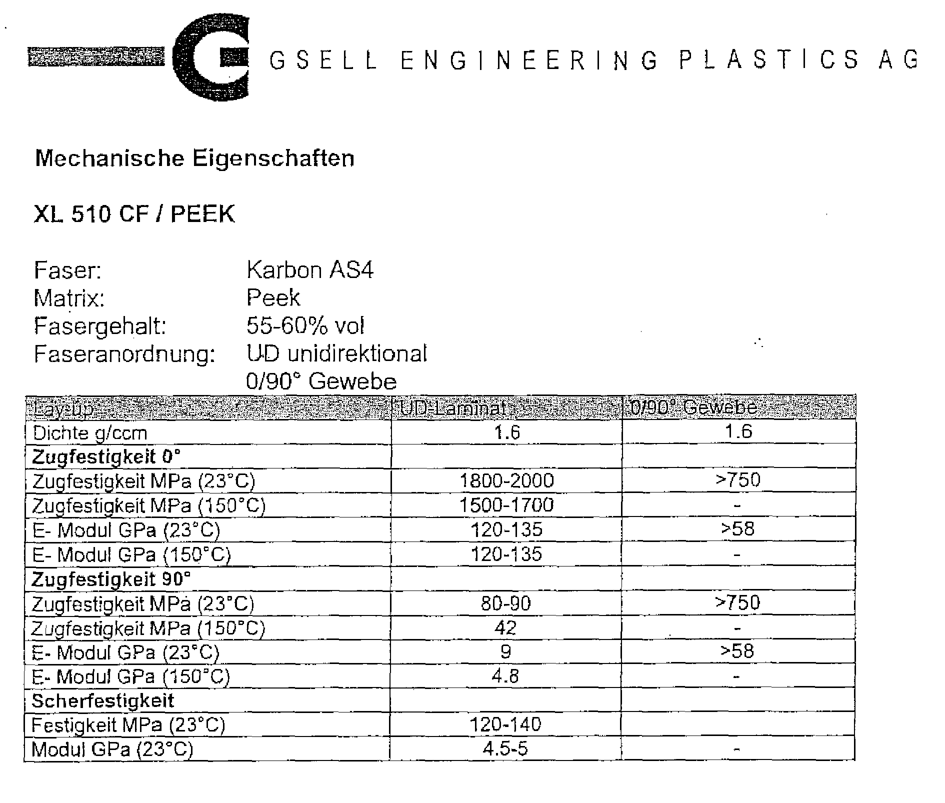

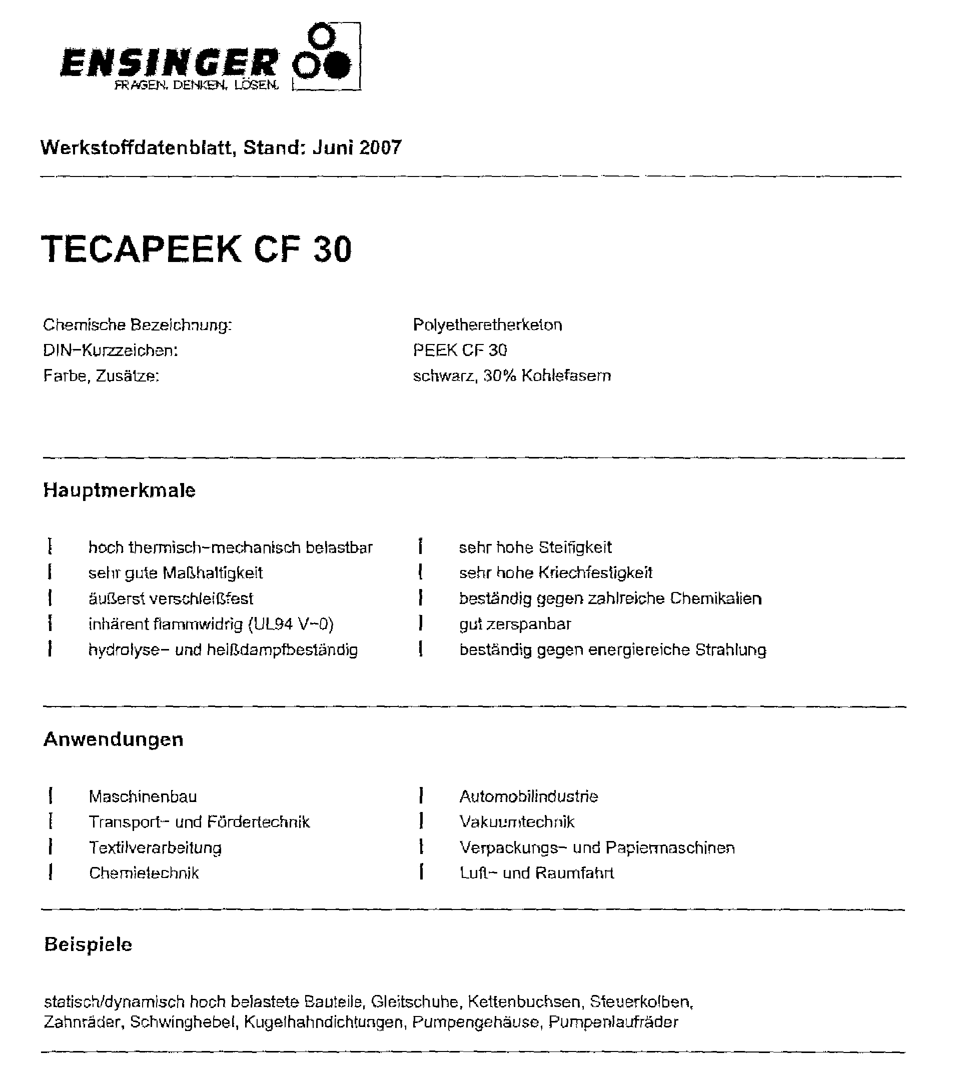

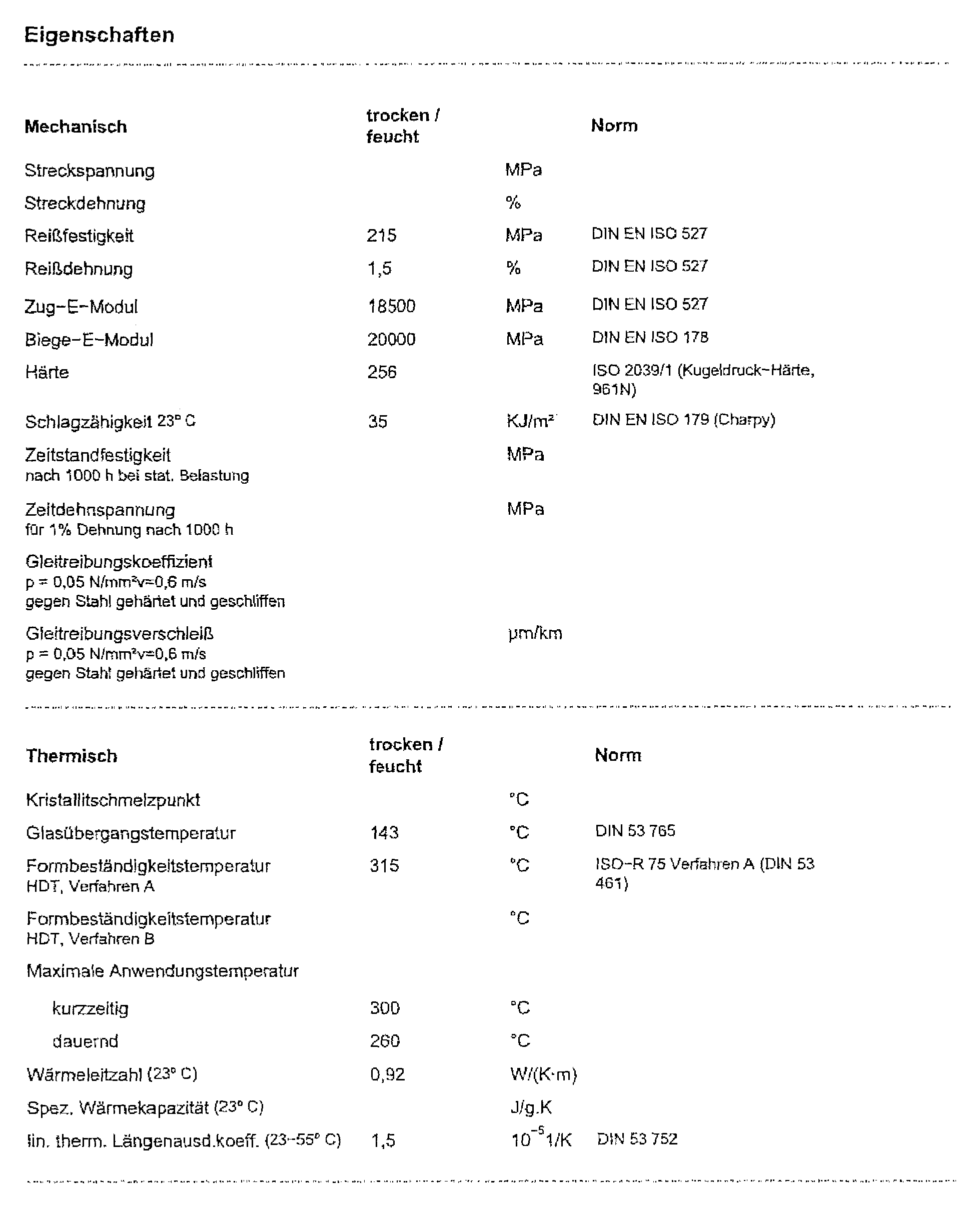

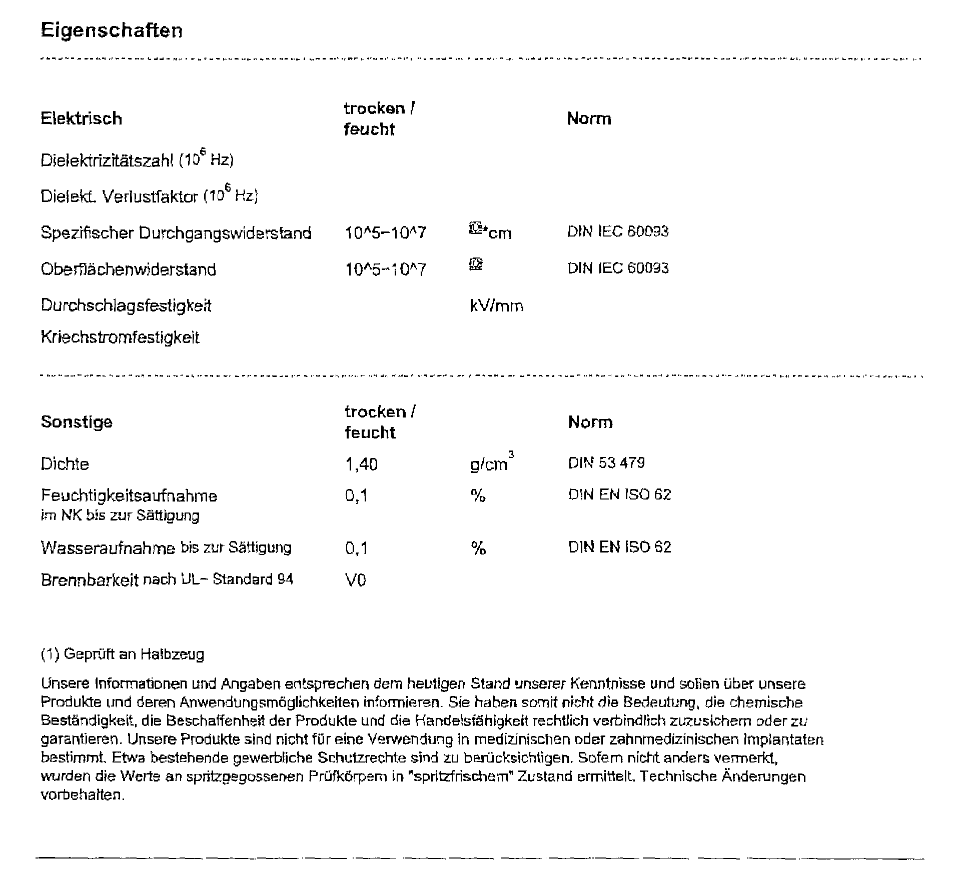

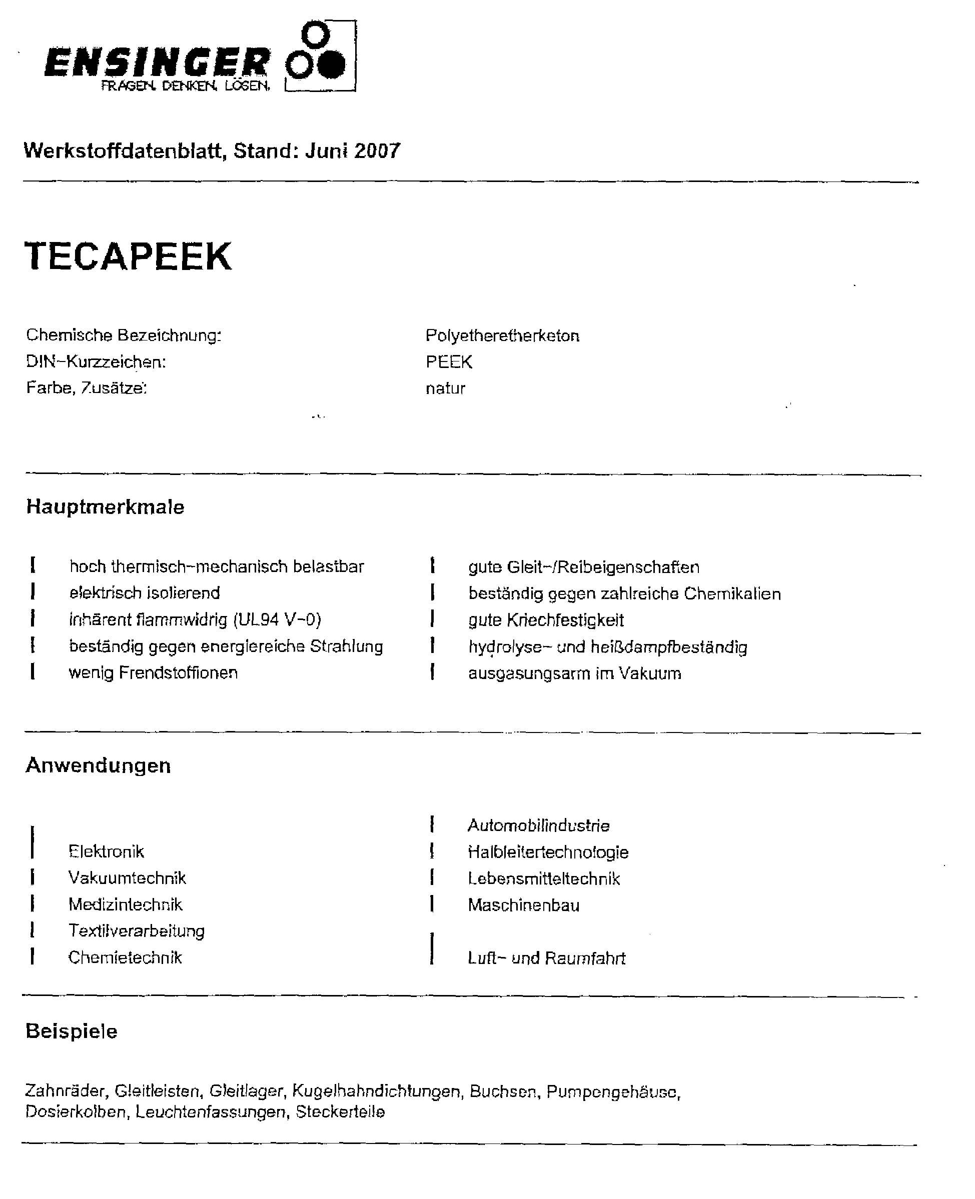

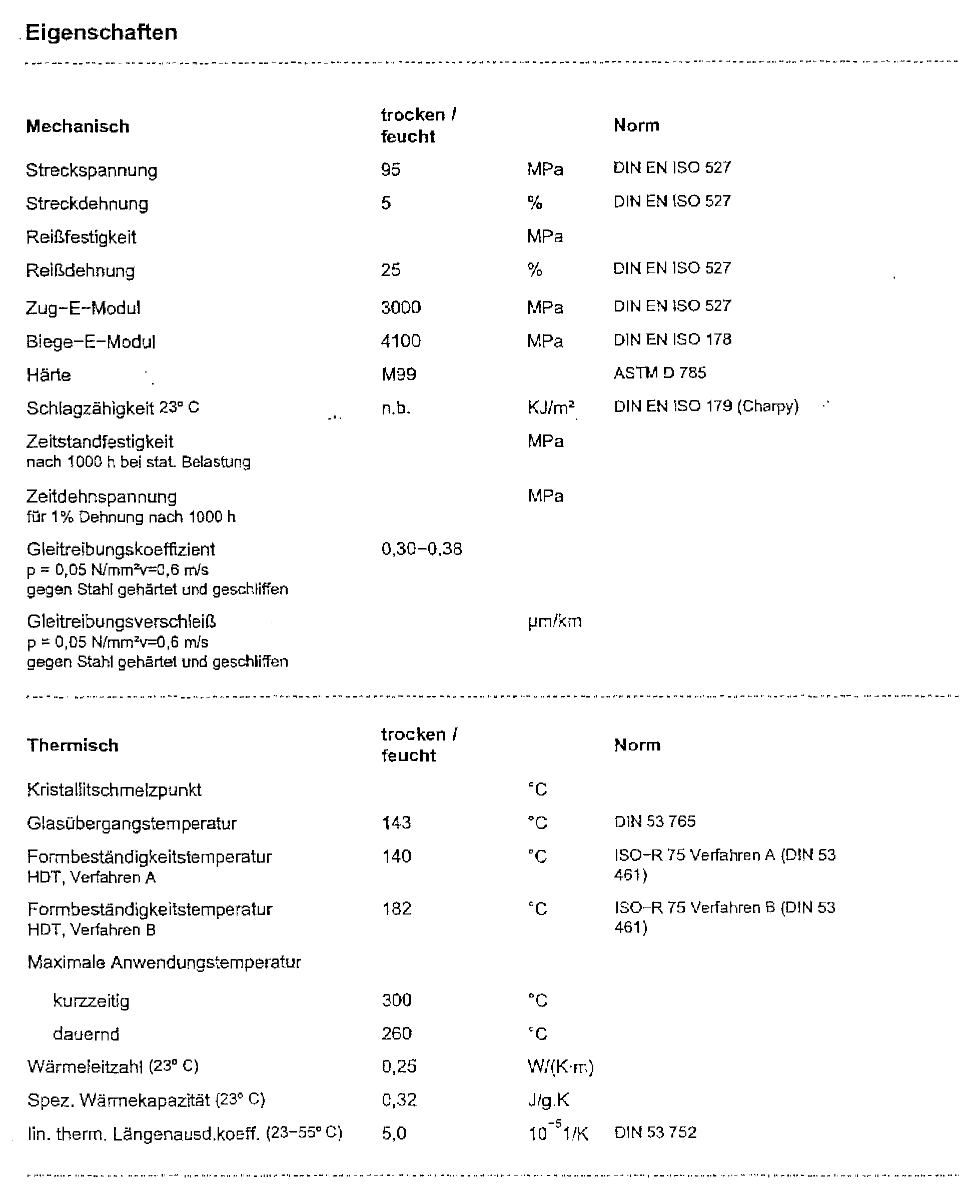

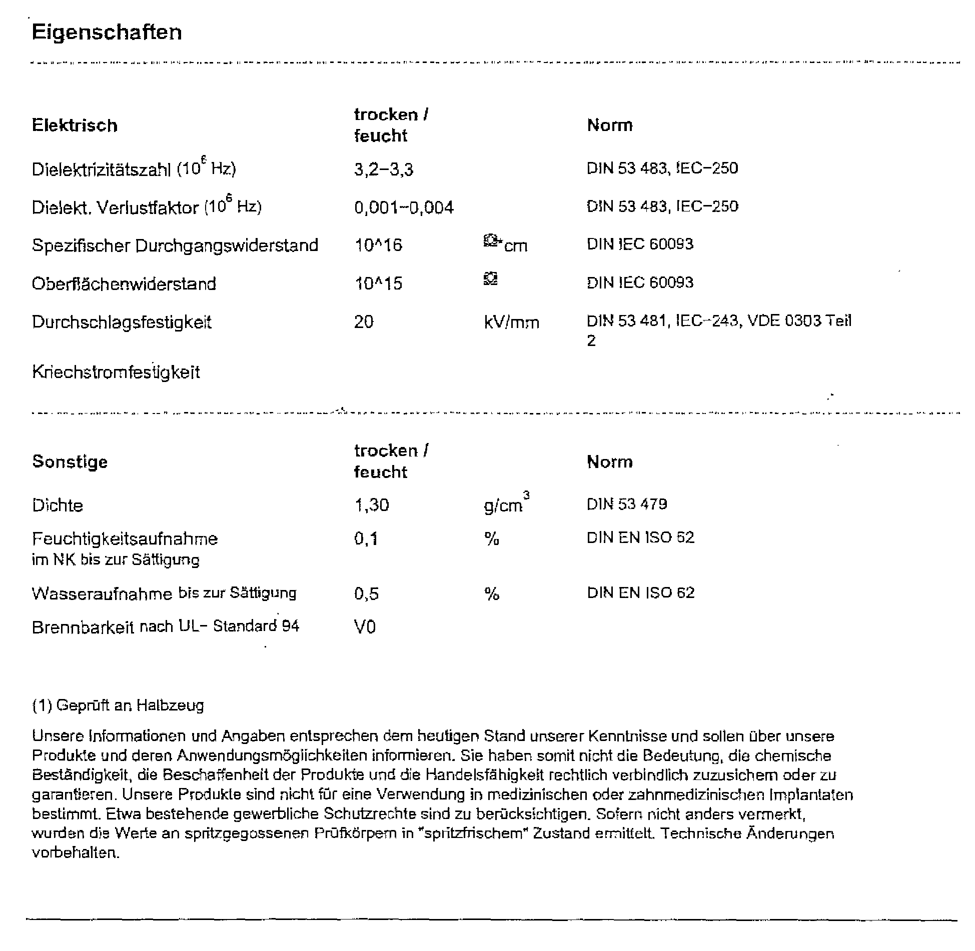

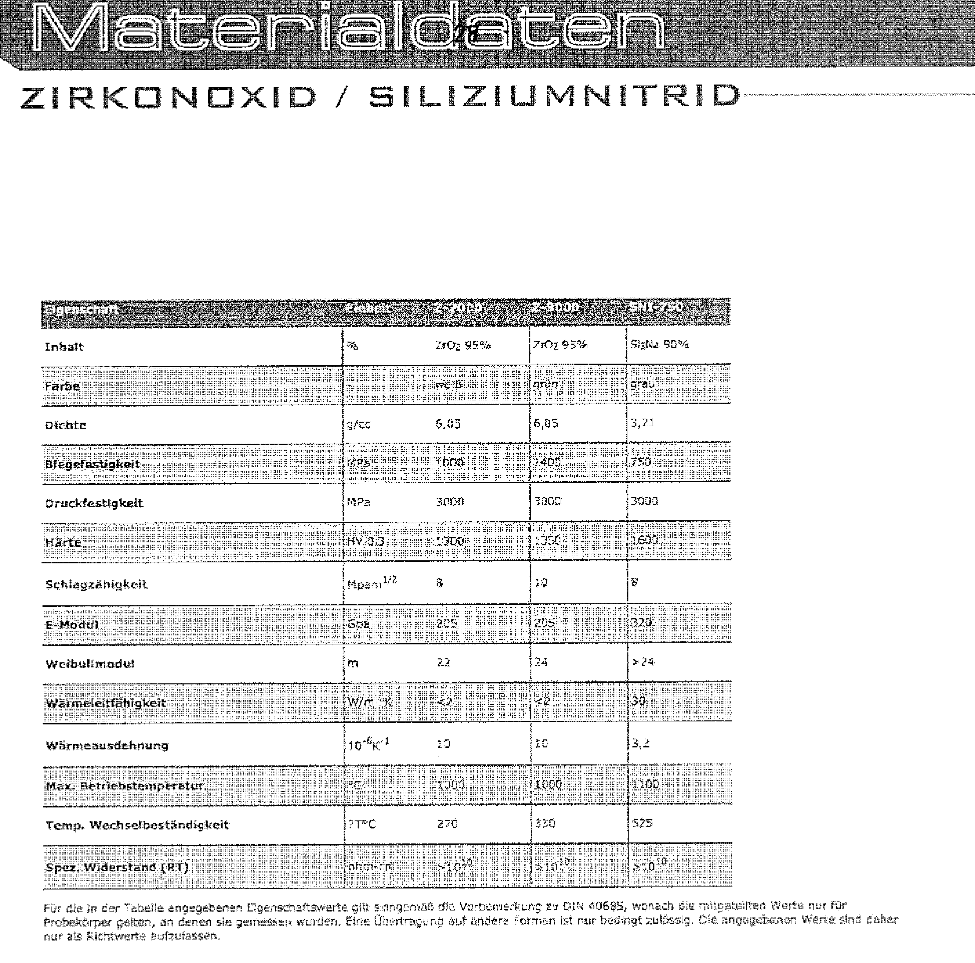

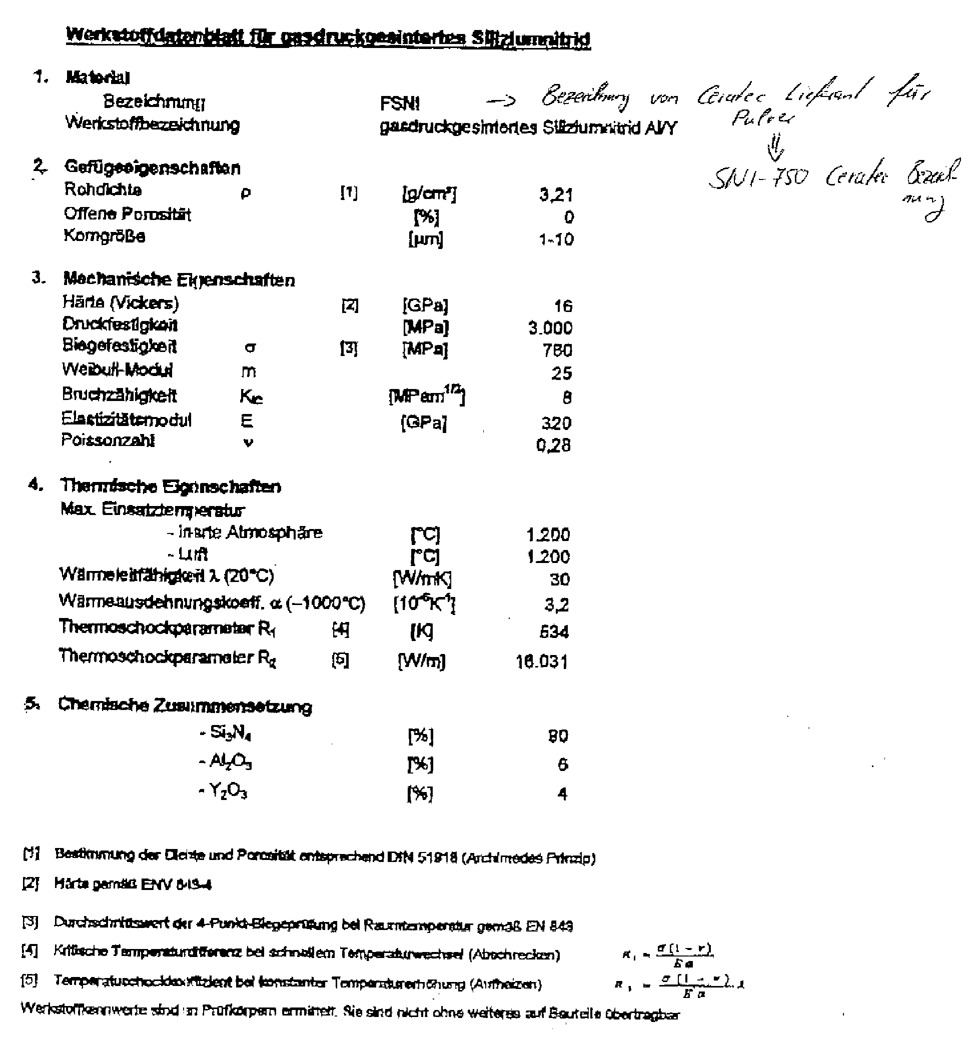

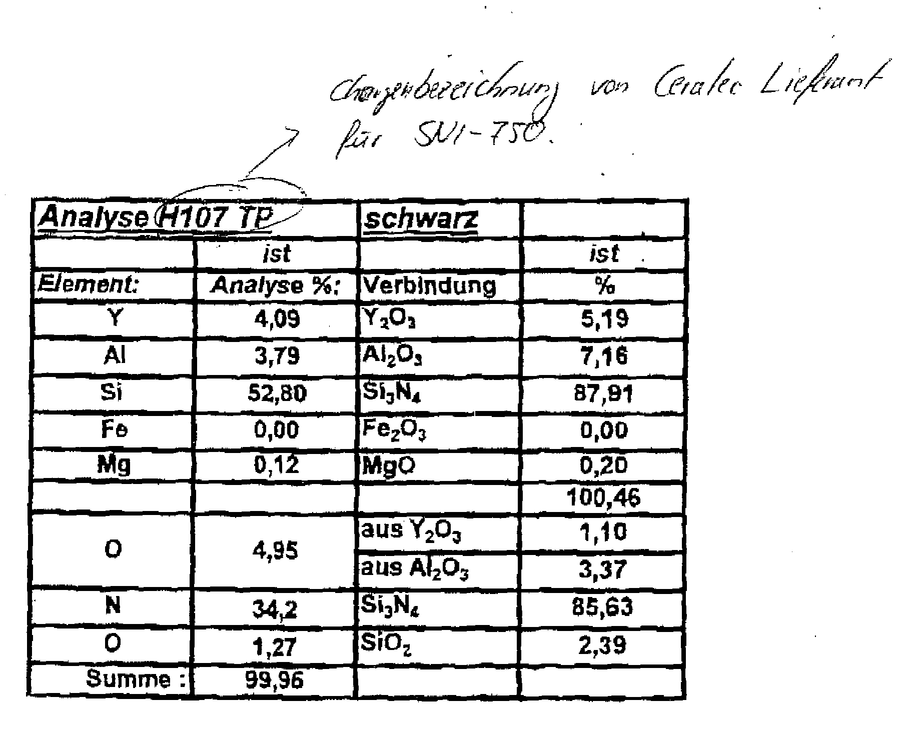

## Patentansprüche

1. Befestigungsmittel (10) für medizinische Zwecke zum Befestigen einer Referenzgeometrie (4) für navigationsunterstützte Operationen an einem Körper, insbesondere an einem Knochen,
wobei wenigstens ein Teil des Befestigungsmittels (10) aus einer karbonfaserverstärkten unsubstituierten, karbonfaserverstärkten einfach substituierten oder karbonfaserverstärkten mehrfach substituierten Polyether-Verbindung, insbesondere aus einer karbonfaserverstärkten unsubstituierten, karbonfaserverstärkten einfach substituierten oder karbonfaserverstärkten mehrfach substituierten aromatischen Polyether-Verbindung besteht,
wobei das Befestigungsmittel (10) in Form einer Klemme (10) ausgestaltet ist,
wobei die Klemme (10) einen Klemmbereich und einen Trägerbereich mit einem Übertragungsabschnitt aufweist, wobei der Klemmbereich und der Übertragungsabschnitt miteinander in Kontakt stehen,
**dadurch gekennzeichnet, dass**
der Übertragungsabschnitt keilförmig ausgebildet ist.

2. Befestigungsmittel (10) gemäß Anspruch 1, wobei es sich bei der karbonfaserverstärkten unsubstituierten, karbonfaserverstärkten einfach substituierten oder karbonfaserverstärkten mehrfach substituierten Polyether-Verbindung um karbonfaserverstärktes unsubstituiertes, karbonfaserverstärktes einfach substituiertes oder karbonfaserverstärktes mehrfach substituiertes Poly(etheretherketon) (PEEK) 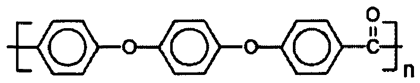 handelt.

3. Befestigungsmittel (10) gemäß Anspruch 1, wobei es sich bei der karbonfaserverstärkten unsubstituierten, karbonfaserverstärkten einfach substituierten oder karbonfaserverstärkten mehrfach substituierten Polyether-Verbindung um karbonfaserverstärktes unsubstituiertes, karbonfaserverstärktes einfach substituiertes oder karbonfaserverstärktes mehrfach substituiertes Poly(etherketon) (PEK) 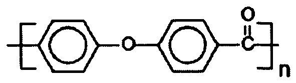 handelt.

4. Befestigungsmittel (10) gemäß Anspruch 1, wobei es sich bei der karbonfaserverstärkten unsubstituierten, karbonfaserverstärkten einfach substituierten oder karbonfaserverstärkten mehrfach substituierten Polyether-Verbindung um karbonfaserverstärktes unsubstituiertes, karbonfaserverstärktes einfach substituiertes oder karbonfaserverstärktes mehrfach substituiertes Poly(etherketonketon) (PEKK) 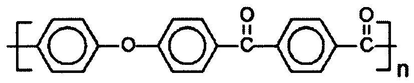 handelt.

5. Befestigungsmittel (10) gemäß Anspruch 1, wobei es sich bei der karbonfaserverstärkten unsubstituierten, karbonfaserverstärkten einfach substituierten oder karbonfaserverstärkten mehrfach substituierten Polyether-Verbindung um karbonfaserverstärktes unsubstituiertes, karbonfaserverstärktes einfach substituiertes oder karbonfaserverstärktes mehrfach substituiertes Polyetherimid (PEI) handelt.

6. Befestigungsmittel (10) gemäß einem der Ansprüche 1 bis 5, wobei wenigstens ein Teil des aus der karbonfaserverstärkten unsubstituierten, karbonfaserverstärkten einfach substituierten oder karbonfaserverstärkten mehrfach substituierten Polyether-Verbindung bestehenden Teiles, insbesondere wenigstens ein Teil des aus karbonfaserverstärktem unsubstituierten, karbonfaserverstärktem einfach substituierten oder karbonfaserverstärktem mehrfach substituierten PEEK, PEK, PEKK oder PEI bestehenden Teiles, eine Karbonfaserverstärkung mittels Langfasern oder Endlosfasern aufweist.

7. Befestigungsmittel (10) gemäß Anspruch 6, wobei der Karbonfasergehalt des wenigstens einen Teiles, das mittels Langfasern oder Endlosfasern verstärkt ist, 50 - 65% vol, bevorzugt 55 - 60% vol beträgt.

8. Befestigungsmittel (10) gemäß einem der Ansprüche 1 bis 7, wobei wenigstens ein Teil des aus der karbonfaserverstärkten unsubstituierten, karbonfaserverstärkten einfach substituierten oder karbonfaserverstärkten mehrfach substituierten Polyether-Verbindung bestehenden Teiles, insbesondere wenigstens ein Teil des aus karbonfaserverstärktem unsubstituierten, karbonfaserverstärktem einfach substituierten oder karbonfaserverstärktem mehrfach substituierten PEEK, PEK, PEKK oder PEI bestehenden Teiles, eine Karbonfaserverstärkung mittels Kurzfasern aufweist.

9. Befestigungsmittel (10) gemäß Anspruch 8, wobei der Karbonfasergehalt des wenigstens einen Teiles, das mittels Kurzfasern verstärkt ist, 25 - 35% vol, bevorzugt 30% vol beträgt.

10. Befestigungsmittel (10) gemäß einem der Ansprüche 1 bis 9, wobei wenigstens ein weiterer Teil aus einer nicht-karbonfaserverstärkten unsubstituierten, nicht-karbonfaserverstärkten einfach substituierten oder nicht-karbonfaserverstärkten mehrfach substituierten Polyether-Verbindung, insbesondere aus nicht-karbonfaserverstärktem unsubstituierten, nicht-karbonfaserverstärktem einfach substituierten oder nicht-karbonfaserverstärktem mehrfach substituierten PEEK, PEK, PEKK oder PEI, besteht.

11. Befestigungsmittel (10) gemäß Anspruch 1, wobei die Klemme (10) an einem Knochen spannungsbalanciert anbringbar ist.

12. Befestigungsmittel (10) gemäß Anspruch 11,
• wobei der Klemmbereich angepasst ist, um die Klemme (10) an einem Knochen zu befestigen;
• wobei eine Wechselwirkung zwischen dem Übertragungsabschnitt und dem Klemmbereich bei einem Klemmvorgang spannungsbalanciert erfolgt.

13. Befestigungsmittel (10) gemäß Anspruch 12,
• wobei der Trägerbereich des weiteren einen Lagerabschnitt aufweist;
• wobei der Klemmbereich zwei Schenkel (1) aufweist, die jeweils drehbar um eine Achse gelagert sind, die jeweils durch den Lagerabschnitt hindurchgeführt ist; und
• wobei der Übertragungsabschnitt mit beiden Schenkeln (1) zugleich symmetrisch in Wechselwirkung tritt und dadurch eine Rotation der Schenkel (1) um ihre Rotationsachsen von gleichem Betrage, aber in entgegen gesetzter Drehrichtung bewirkt; so dass die Klemme am Knochen spannungsbalanciert anbringbar ist.

14. Befestigungsmittel (10) gemäß Anspruch 13,
• wobei der Lagerabschnitt eine Hauptachse aufweist; und
• wobei das Befestigungsmittel (10) so ausgebildet ist, dass der keilförmige Übertragungsabschnitt entlang der Hauptachse des Lagerabschnittes sich bewegt und dabei die Schenkel (1) des Klemmbereiches in Rotation versetzt.

15. Befestigungsmittel (10) gemäß Anspruch 14, wobei der keilförmige Übertragungsabschnitt eine Führung für jeweils ein Ende der Schenkel (1) aufweist.

16. Befestigungsmittel (10) gemäß einem der Ansprüche 14 bis 15, wobei der Trägerabschnitt einen Betätigungsabschnitt aufweist, der bei Betätigung den Übertragungsabschnitt in Bewegung versetzt.

17. Befestigungsmittel (10) gemäß einem der Ansprüche 1 bis 16 mit wenigstens einem Eindringkörper (16), der aus einer Siliziumnitrid-Keramik, bevorzugt einer beta-Siliziumnitrid-Keramik, besteht.

18. Befestigungsmittel (10) gemäß einem der Ansprüche 1 bis 17 mit wenigstens einem Eindringkörper (16), der an dem Befestigungsmittel (10) mittels einer Verliersicherung (17a) angebracht ist.

19. Befestigungsmittel (10) gemäß Anspruch 18, wobei die Verliersicherung (17a) ein Plättchen aufweist, durch das der wenigstens eine Eindringkörper (16) teilweise hindurchragt, und wobei das Plättchen einen Teil des Eindringkörpers, der nicht durch das Plättchen hindurchragt, zwischen dem Befestigungsmittel (10) und dem Plättchen einklemmt.

20. Befestigungsmittel (10) gemäß einem der Ansprüche 18 bis 19, wobei die Verliersicherung (17a) mittels eines Klickverschlusses an dem Befestigungsmittel (10) befestigbar ist.

21. System umfassend
• ein Befestigungsmittel (10) gemäß einem der Ansprüche 1 bis 20; und
• eine Referenzgeometrie (4) für navigationsunterstützte Operationen.

## Claims

1. An attachment means (10) for medical purposes for attaching a reference geometry (4) for navigation-assisted operations to a body, in particular a bone,
wherein at least a part of the attachment means (10) consists of a carbon fibre-reinforced unsubstituted, carbon fibre-reinforced singly substituted or carbon fibre-reinforced multiply substituted polyether compound, in particular a carbon fibre-reinforced unsubstituted, carbon fibre-reinforced singly substituted or carbon fibre-reinforced multiply substituted aromatic polyether compound,
wherein the attachment means (10) is formed as a clamp,
wherein the clamp (10) has a clamping region and a carrier region with a transmission section, wherein the clamping region and the transmission region are in contact with each other,
**characterised in that**
the transmission section is formed in the shape of a wedge.

2. The attachment means (10) according to claim 1, wherein the carbon fibre-reinforced unsubstituted, carbon fibre-reinforced singly substituted or carbon fibre-reinforced multiply substituted polyether compound is carbon fibre-reinforced unsubstituted, carbon fibre-reinforced singly substituted or carbon fibre-reinforced multiply substituted poly(etheretherketone) (PEEK)

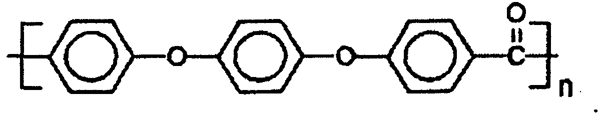

3. The attachment means (10) according to claim 1, wherein the carbon fibre-reinforced unsubstituted, carbon fibre-reinforced singly substituted or carbon fibre-reinforced multiply substituted polyether compound is carbon fibre-reinforced unsubstituted, carbon fibre-reinforced singly substituted or carbon fibre-reinforced multiply substituted poly(etherketone) (PEK)

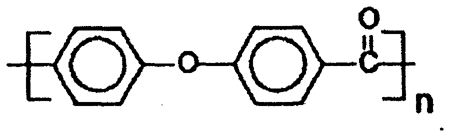

4. The attachment means (10) according to claim 1, wherein the carbon fibre-reinforced unsubstituted, carbon fibre-reinforced singly substituted or carbon fibre-reinforced multiply substituted polyether compound is carbon fibre-reinforced unsubstituted, carbon fibre-reinforced singly substituted or carbon fibre-reinforced multiply substituted poly(ketoneketone) (PEKK)

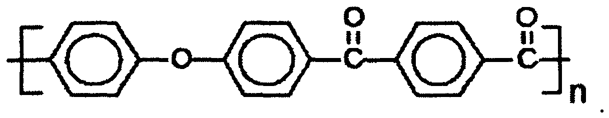

5. The attachment means (10) according to claim 1, wherein the carbon fibre-reinforced unsubstituted, carbon fibre-reinforced singly substituted or carbon fibre-reinforced multiply substituted polyether compound is carbon fibre-reinforced unsubstituted, carbon fibre-reinforced singly substituted or carbon fibre-reinforced multiply substituted polyetherimide (PEI).

6. The attachment means (10) according to any one of claims 1 to 5, wherein at least a part of the part consisting of the carbon fibre-reinforced unsubstituted, carbon fibre-reinforced singly substituted or carbon fibre-reinforced multiply substituted polyether compound, in particular at least a part of the part consisting of the carbon fibre-reinforced unsubstituted, carbon fibre-reinforced singly substituted or carbon fibre-reinforced multiply substituted PEEK, PEK; PEKK or PEI, has a carbon fibre reinforcement using long fibres or endless fibres.

7. Attachment means (10) according to claim 6, wherein the carbon fibre contents of the at least one part reinforced by long fibres or endless fibres is 50 - 65 % vol, preferably 55 - 60 % vol.

8. The attachment means (10) according to any one of claims 1 to 5, wherein at least a part of the part consisting of the carbon fibre-reinforced unsubstituted, carbon fibre-reinforced singly substituted or carbon fibre-reinforced multiply substituted polyether compound, in particular at least a part of the part consisting of the carbon fibre-reinforced unsubstituted, carbon fibre-reinforced singly substituted or carbon fibre-reinforced multiply substituted PEEK, PEK; PEKK or PEI, has a carbon fibre reinforcement using short fibres.

9. The attachment means (10) according to claim 8, wherein the carbon fibre contents of the at least one part reinforced by short fibres is 25 - 35 % vol, preferably 30 % vol.

10. The attachment means (10) according to any one of claims 1 to 9, wherein at least one further part consists of a non-carbon fibre-reinforced unsubstituted, non-carbon fibre-reinforced singly substituted or non-carbon fibre-reinforced multiply substituted polyether compound, in particular of non-carbon fibre-reinforced unsubstituted, non-carbon fibre-reinforced singly substituted or non-carbon fibre-reinforced multiply substituted PEEK, PEK; PEKK or PEI.

11. Attachment means (10) according to claim 1, wherein the clamp (10) is attachable to a bone in a tension-balanced state.

12. The attachment means (10) according to claim 11,
• wherein the clamping region is adapted to attach the clamp to a bone;
• wherein an interaction between the transmission section and the clamping region occurs in a tension-balanced manner.

13. The attachment means (10) according to claim 11,
• wherein the carrier region further has a support section;
• wherein the clamping region has two brackets (1) which are each supported rotatably around an axis which respectively runs through the support section; and
• wherein the transmission section interacts with both brackets (1) simultaneously and symmetrically, whereby it causes a rotation of the brackets (1) around their rotational axes of the same amount but in opposing rotational directions so that the clamp is attachable to the bone in a tension-balanced manner.

14. The attachment means (10) according to claim 13,
• wherein the support section has a main axis; and
• wherein the attachment means (10) is formed such that the wedge-shaped transmission section moves along the main axis of the support section while causing the brackets (1) of the clamping region to rotate.

15. The attachment means (10) according to claim 14, wherein the wedge-shaped transmission section has a guide for each end of the brackets (1).

16. The attachment means (10) according to any one of claims 14 to 15, wherein the support section has an operation section which, if operated, causes the transmission section to move.

17. The attachment means (10) according to any one of claims 14 to 15 with at least one penetrating body (16) consisting of a silicon nitride ceramic, preferably a beta-silicon nitride ceramic.

18. The attachment means (10) according to any one of claims 1 to 17 with at least one penetrating body (16) attached to the attachment means (10) via a retainer and/or self.-locking nut (17a).

19. The attachment means (10) according to claim 18, wherein the retainer (17a) has a platelet through which the at least one penetrating body (16) partially protrudes and wherein the platelet clamps the part of the penetrating body which does not protrude through the platelet between the attachment means (10) and the platelet.

20. The attachment means (10) according to any one of claims 18 to 19, wherein the retainer (19a) can be fastened to the attachment means (10) via a clip fastener and/or quick release.

21. A system, comprising:
• an attachment means (10) according to any one of claims 1 to 20; and
• a reference geometry (4) for navigation-assisted operations.

## Revendications

1. Moyen de fixation (10) pour applications médicales pour fixer une géométrie de référence (4) pour des opérations assistées par navigation au niveau d'un corps, en particulier au niveau d'un os,
où au moins une partie du moyen de fixation (10) est constitué d'un composé polyéther non substitué et renforcé de fibre de carbone, d'un composé polyéther substitué une fois et renforcé de fibre de carbone ou d'un composé polyéther substitué plusieurs fois et renforcé de fibres de carbone, en particulier d'un composé polyéther aromatique non substitué et renforcé de fibre de carbone, d'un composé polyéther aromatique substitué une fois et renforcé de fibre de carbone ou d'un composé polyéther aromatique substitué plusieurs fois et renforcé de fibres de carbone,
où le moyen de fixation (10) est configuré en forme de pince (10),
où la pince (10) comporte une zone de serrage et une zone porteuse avec une partie de transmission, où la zone de serrage et la partie de transmission sont en contact l'une avec l'autre,
**caractérisé en ce que**
la partie de transmission est configurée en forme de coin.

2. Moyen de fixation (10) selon la revendication 1, où le composé polyéther non substitué et renforcé de fibres de carbone, substitué une fois et renforcé de fibres de carbone ou substitué plusieurs fois et renforcé de fibres de carbone est un poly(éther éther cétone) (PEEK) non substitué et renforcé de fibres de carbone, substitué une fois et renforcé de fibres de carbone ou substitué plusieurs fois et renforcé de fibres de carbone.

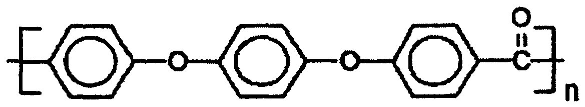

3. Moyen de fixation (10) selon la revendication 1, où le composé polyéther non substitué et renforcé de fibres de carbone, substitué une fois et renforcé de fibres de carbone ou substitué plusieurs et fois renforcé de fibres de carbone est un poly(éther cétone) (PEK) non substitué et renforcé de fibres de carbone, substitué une fois et renforcé de fibres de carbone ou substitué plusieurs fois et renforcé de fibres de carbone.

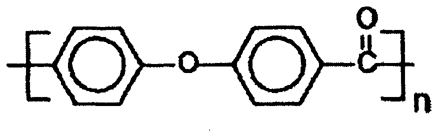

4. Moyen de fixation (10) selon la revendication 1, où le composé polyéther non substitué et renforcé de fibres de carbone, substitué une fois et renforcé de fibres de carbone ou substitué plusieurs fois et renforcé de fibres de carbone est un poly(éther cétone cétone) (PEKK) non substitué et renforcé de fibres de carbone, substitué une fois et renforcé de fibres de carbone ou substitué plusieurs fois et renforcé de fibres de carbone.

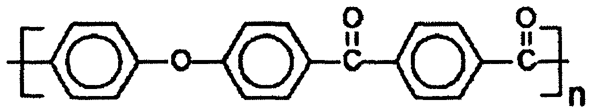

5. Moyen de fixation (10) selon la revendication 1, le composé polyéther non substitué et renforcé de fibres de carbone, substitué une fois et renforcé de fibres de carbone ou substitué plusieurs fois et renforcé de fibres de carbone est un polyétherimide (PEI) non substitué et renforcé de fibres de carbone, substitué une fois et renforcé de fibres de carbone ou substitué plusieurs fois et renforcé de fibres de carbone.

6. Moyen de fixation (10) selon l'une des revendications 1 à 5, où au moins une partie de la partie constituée du composé polyéther non substitué et renforcé de fibres de carbone, substitué une fois et renforcé de fibres de carbone ou substitué plusieurs fois et renforcé de fibres de carbone, en particulier au moins une partie de la partie constituée de PEEK, PEK, PEKK ou PEI non substitués et renforcés de fibres de carbone, substitués une fois et renforcés de fibres de carbone ou substitués plusieurs fois et renforcés de fibres de carbone comporte un renforcement de fibres de carbone au moyen de fibres longues ou de fibres continues.

7. Moyen de fixation (10) selon la revendication 6, où la teneur en fibre de carbone de l'au moins une partie, renforcée au moyen de fibres longues ou de fibres continues, est de 50 - 65 % vol, de préférence de 55 - 60 % vol.

8. Moyen de fixation (10) selon l'une des revendications 1 à 7, où au moins une partie de la partie constituée du composé polyéther non substitué et renforcé de fibres de carbone, substitué une fois et renforcé de fibres de carbone ou substitué plusieurs fois et renforcé de fibres de carbone, en particulier au moins une partie de la partie constituée de PEEK, PEK, PEKK ou PEI non substitués et renforcés de fibres de carbone, substitués une fois et renforcés de fibres de carbone ou substitués plusieurs fois et renforcés de fibres de carbone comporte un renforcement de fibres de carbone au moyen de fibres courtes.

9. Moyen de fixation (10) selon la revendication 8, où la teneur en fibre de carbone de l'au moins une partie, renforcée au moyen de fibres courtes, est de 25 - 30 % vol, de préférence de 30 % vol.

10. Moyen de fixation (10) selon l'une des revendications 1 à 9, où au moins une autre partie est constituée d'un composé polyéther non substitué et non renforcé de fibres de carbone, substitué une fois et non renforcé de fibres de carbone ou substitué plusieurs fois et non renforcé de fibres de carbone, en particulier constituée de PEEK, PEK, PEKK ou PEI non substitués et non renforcés de fibres de carbone, substitués une fois et non renforcés de fibres de carbone ou substitués plusieurs fois et non renforcés de fibres de carbone.

11. Moyen de fixation (10) selon la revendication 1, où la pince (10) est apte à être attachée à un os d'une manière préservant l'équilibre des forces.

12. Moyen de fixation (10) selon la revendication 11,
• où la zone de serrage est adaptée pour fixer la pince (10) sur un os ;
• où une interaction entre la partie de transmission et la zone de serrage lors d'un processus de serrage se fait d'une manière préservant l'équilibre des forces.

13. Moyen de fixation (10) selon la revendication 12,
• où la zone porteuse comporte en outre une partie de palier ;
• où la zone de serrage comporte deux branches (1), respectivement montées en rotation autour d'un axe qui traverse respectivement la partie de palier ;et
• où la partie de transmission interagit avec les deux branches (1) simultanément et symétriquement et provoque ainsi une rotation des branches (1) autour de leur axe de rotation, de même quantité mais dans des directions de rotation opposées ; de sorte que la pince est apte à être attachée sur l'os d'une manière préservant l'équilibre des forces.

14. Moyen de fixation (10) selon la revendication 13,
• où la partie de palier comporte un axe principal ; et
• où le moyen de fixation (10) est configuré de telle manière que la partie de transmission en forme de coin se déplace le long de l'axe principal de la partie de palier et ainsi met les branches (1) de la zone de serrage en rotation.

15. Moyen de fixation (10) selon la revendication 14, où la partie de transmission en forme de coin comporte un guidage pour respectivement une extrémité des branches (1).

16. Moyen de fixation (10) selon l'une des revendications 14 à 15, où la partie porteuse comporte une partie d'actionnement qui met en mouvement la partie de transmission lorsqu'elle est actionnée.

17. Moyen de fixation (10) selon l'une des revendication 1 à 16 avec au moins un corps de pénétration (16) constitué d'une céramique en nitrure de silicium, de préférence d'une céramique en nitrure de silicium beta.

18. Moyen de fixation (10) selon l'une des revendications 1 à 17 avec au moins un corps de pénétration (16) attaché sur le moyen de fixation (10) au moyen d'une sécurité anti-perte (17a).

19. Moyen de fixation (10) selon la revendication 18, où la sécurité anti-perte (17a) comporte une plaquette à travers laquelle passe partiellement l'au moins un corps de pénétration (16) et où la plaquette coince une partie du corps de pénétration qui ne traverse pas la plaquette entre le moyen de fixation (10) et la plaquette.

20. Moyen de fixation (10) selon l'une des revendications 18 à 19, où la sécurité anti-perte (17a) est apte à être attachée au moyen de fixation (10) au moyen d'une fermeture à cliquet.

21. Système comprenant
• un moyen de fixation (10) selon l'une des revendications 1 à 20 ; et
• une géométrie de référence (4) pour des opérations assistées par navigation.
